(19) 

Europäisches
Patentamt
European
Patent Office
Office européen
des brevets

(11) **EP 4 082 574 A1**

(12) **EUROPEAN PATENT APPLICATION**
published in accordance with Art. 153(4) EPC

(43) Date of publication:
**02.11.2022 Bulletin 2022/44**

(21) Application number: **20906248.8**

(22) Date of filing: **25.12.2020**

(51) International Patent Classification (IPC):
*A61K 45/00* (2006.01)  *A61K 39/395* (2006.01)
*A61P 25/02* (2006.01)  *A61P 25/04* (2006.01)
*A61P 27/02* (2006.01)  *C07K 16/28* (2006.01)
*C07K 16/46* (2006.01)  *C12N 15/13* (2006.01)

(52) Cooperative Patent Classification (CPC):
**A61K 39/395; A61K 45/00; A61P 25/02;**
**A61P 25/04; A61P 27/02; C07K 16/00;**
**C07K 16/28; C07K 16/46**

(86) International application number:
**PCT/JP2020/049004**

(87) International publication number:
**WO 2021/132673 (01.07.2021 Gazette 2021/26)**

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(30) Priority: **26.12.2019 JP 2019236661**

(71) Applicants:
• **OSAKA UNIVERSITY**
**Suita-shi**
**Osaka 565-0871 (JP)**
• **Mitsubishi Tanabe Pharma Corporation**
**Osaka-shi,**
**Osaka 541-8505 (JP)**

(72) Inventors:
• **YAMASHITA, Toshihide**
**Suita-shi, Osaka 565-0871 (JP)**

• **ITOKAZU, Takahide**
**Suita-shi, Osaka 565-0871 (JP)**
• **KATAOKA, Hirotoshi**
**Osaka-shi, Osaka 541-8505 (JP)**
• **HIRATA, Takeshi**
**Osaka-shi, Osaka 541-8505 (JP)**
• **IWAMOTO, Shosuke**
**Osaka-shi, Osaka 541-8505 (JP)**
• **SASAKI, Atsushi**
**Osaka-shi, Osaka 541-8505 (JP)**

(74) Representative: **Hoffmann Eitle**
**Patent- und Rechtsanwälte PartmbB**
**Arabellastraße 30**
**81925 München (DE)**

Remarks:
The complete document including Reference
Table(s) and the Sequence Listing(s) can be
downloaded from the EPO website

(54) **AGENT FOR PREVENTING OR TREATING ACUTE-PHASE NEUROMYELITIS OPTICA**

(57) This invention provides an agent for preventing or treating acute phase neuromyelitis optica, and pain symptoms in neuromyelitis optica, which comprises a RGMa inhibiting substance.

EP 4 082 574 A1

**(Cont. next page)**

**Description**

TECHNICAL FIELD

The present invention relates to an agent for preventing or treating acute phase neuromyelitis optica comprising a RGMa inhibiting substance, and an agent for preventing or treating pain symptoms in neuromyelitis optica.

BACKGROUND ART

Neuromyelitis optica (NMO), also known as Devic disease, is an inflammatory central nervous system disease characterized by severe neuritis optica and transverse myelitis extending over 3 or more cones. In 2004, an IgG (NMO-IgG) specific to neuromyelitis optica was discovered (Non Patent Document 1), and it has been reported that aquaporin-4 (AQP4) which is a water channel protein densely expressed in the foot processes of astrocytes is a target antigen of the IgG, namely NMO-IgG is an anti-AQP4 antibody (Non Patent Document 2).

Therefore, neuromyelitis optica is, an astrocytopathy in which astrocytes are destructed by anti-AQP4 antibodies, unlike multiple sclerosis, a demyelinating disease. In the pathological conditions of neuromyelitis optica, inflow of an anti-AQP4 antibody into the brain and spinal cord and complement activation occur due to central nervous system inflammation caused by activation of cellular immunity and hyperpermeability of blood spinal cord barrier (BSCB), or blood brain barrier (BBB) in the case of the brain, and therefore it is conceivable that loss of AQP4, or destruction and dysfunction of astrocytes is induced extensively. It is also believed that inflammatory cell infiltration, demyelination or axonopathy, and phagocytosis or softening of tissue by macrophages/microglia secondarily occur due to functional disturbances, such as cellular dysfunction (complement-dependent, or antibody-dependent cellular dysfunction) of astrocytes, disruption of the blood brain barrier or abnormality of glutamate metabolism caused by astrocyte loss, and in consequence necrosis of nerve tissues is induced (Non Patent Documents 3, and 4).

The criteria of Wingerchuk, *et al.* published in 2006 have been widely used as the typical diagnostic criteria for neuromyelitis optica (Non Patent Document 5), while neuritis optica (recurrent or bilateral), and cases solely involving myelitis as long as three or more vertebrae were also understood as the diseases in the same category of neuromyelitis optica spectrum disorder (NMOSD) in addition to typical neuromyelitis optica since 2007 (Non Patent Document 6). In other words, the recent disease concept of neuromyelitis optica is considered to be broader than the conventional concept of mere neuromyelitis optica. Thus, it is difficult to diagnose neuromyelitis optica solely based on clinical and imaging findings, such as length of spinal cord lesion, and therefore examination of an anti-AQP4 antibody is extremely important in diagnosing neuromyelitis optica, and determining the treatment policy thereon.

The symptoms of acute phase neuromyelitis optica are often more severe than those of multiple sclerosis, and a single recurrence of neuritis optica may cause blindness, and myelitis may confine a patient to a wheelchair. Therefore, it is important to begin treatment promptly. In addition, since the onset of neuromyelitis optica is associated with severe pain in both the acute phase and chronic phase, it is important to conduct a treatment for alleviating the pain at the same time.

Steroid pulse therapy is the first choice for the treatment of acute phase neuromyelitis optica (Non Patent Document 7), and its efficacy has been widely recognized in clinical practice. Since for multiple sclerosis, the therapy has a proven effect of promoting recovery from exacerbation of clinical symptom in the acute phase, it is expected that the same effect can be obtained on neuromyelitis optica (Non Patent Documents 8 to 10). According to the standard method of the steroid pulse therapy, which is similar to the treatment method for multiple sclerosis, 1,000 mg/day of methylprednisolone is administered by instillation for 3 consecutive days, and if symptom improvement is inadequate, one or two cycles are additionally repeated. When the treatment effect of steroid pulse therapy turns out to be poor, it is preferable to actively consider plasma exchange therapy as the second-line treatment (Non Patent Documents 11 and 12), and in a severe case it is preferable to begin the plasma exchange therapy in an early stage.

As described above, currently with respect to a treatment for the acute phase, there is only an accumulation of case reports based on the potential of therapies that have been shown to be useful for multiple sclerosis or neuromyelitis optica. In addition, there is currently no effective therapeutic agent for acute phase neuromyelitis optica on the market.

RGM (repulsive guidance molecule) is a membrane protein that has been initially identified as an axon guidance molecule in the visual system (see Non Patent Document 13). RGM family includes three members called RGMa, RGMb, and RGMc (Non Patent Document 14). At least RGMa and RGMb are known to work in the same signaling mechanism (Non Patent Document 15). RGMc plays an important role in iron metabolisms.

Subsequent studies have revealed that RGM functions to control, for example, axon guidance and laminar formation in *Xenopus* and chick embryos, and cephalic neural tube closure in mouse embryos (Non Patent Document 16). Patent Document 1 discloses an axon regeneration promoting agent containing an anti-RGM neutralizing antibody as an active ingredient.

In addition to its functions in developmental stages, RGMa is expressed again after central nervous system injuries in an adult human and rat. Further, inhibition of RGMa in rat promotes axon growth after spinal cord injuries and facilitates functional recovery (Non-Patent Document 17). From these facts, RGMa is considered as an inhibitor of axon regeneration after central nervous system injuries. Specific examples of antibodies to neutralize RGMa include those described in Patent Document 2 (such as 5F9, and 8D1), Patent Document 3 (such as AE12-1, an AE12-1Y), and Patent Document 4 (such as r116A3, r70E4, r116A3C, and rH116A3).

It has also been known that anti-RGMa neutralizing antibodies are effective for inhibiting onset of neuromyelitis optica (Non-Patent Document 18).

As described above, roles of RGMa in central nervous system injuries have been reported, and its effect on onset inhibition of neuromyelitis optica has been suggested, however involvement of RGMa in the treatment of neuromyelitis optica, especially in the acute phase, has not been identified and no such therapeutic agent has been known.

CITATION LIST

Patent Document

[Patent Document 1] International Publication No. WO2005/087268
[Patent Document 2] International Publication No. WO2009/106356
[Patent Document 3] International Publication No. WO2013/112922
[Patent Document 4] International Publication No. WO2016/175236

Non Patent Document

[Non Patent Document 1] Lancet, 364: 2106-2112, 2004
[Non Patent Document 2] J Exp Med, 202: 473-477, 2005
[Non Patent Document 3] Jpn. J. Clin. Immunol., 35(2), 129-135 (2012)
[Non Patent Document 4] Journal of the Neurological Sciences, 306 (2011) 183-187
[Non Patent Document 5] Neurology, 66: 1485-1489, 2006
[Non Patent Document 6] Lancet Neurol, 6: 805-815, 2007
[Non Patent Document 7] Curr Treat Options Neurol, 12: 244-255, 2010
[Non Patent Document 8] Neurology, 53: 1107-1114, 1999
[Non Patent Document 9] Magn Reson Med Sci, 7: 55-58, 2008
Non Patent Document 10] Tohoku J Exp Med, 215: 55-59, 2008
[Non Patent Document 11] Neurology, 63: 1081-1083, 2004
[Non Patent Document 12] Mult Sclr, 13: 128-132, 2007
[Non Patent Document 13] Neuron, 5, 735-743 (1990).
[Non Patent Document 14] Philos. Trans. R. Soc. Lond. B Biol. Sci., 361: 1513-29, 2006
[Non Patent Document 15] Biochem. Biophys. Res. Commun., 382, 795-800 (2009).
[Non Patent Document 16] Curr. Opin. Neurobiol., 17, 29-34 (2007).
[Non Patent Document 17] J. Cell Biol., 173, 47-58 (2006).
[Non Patent Document 18] Scientific Reports, 8: 34 1-9 (2018)

SUMMARY OF INVENTION

Problem to be solved by the invention

The inhibitory effect of an anti-RGMa neutralizing antibody on the onset of neuromyelitis optica is described in Non Patent Document 18. In this document, it is reported that a positive effect on inhibition of the onset of neuromyelitis optica is obtained by direct injection of an IgG from anti AQP4 antibody-positive NMO patient to the spinal cord of a healthy animal with simultaneous administration of an anti-RGMa neutralizing antibody. However, there is no report about the effect of an anti-RGMa neutralizing antibody on a model of acute phase neuromyelitis optica reflecting human pathological conditions, and moreover it is not clear solely from the disclosure of this document, whether the anti-RGMa neutralizing antibody exhibits a preventive or therapeutic effect on acute neuromyelitis optica.

In addition, since onset of neuromyelitis optica is associated with severe pain, an agent for preventing or treating neuromyelitis optica capable of mitigating or treating the pain symptoms associated with the disease is desperately demanded.

An object of the present invention is to provide a drug effective on the pathology and symptoms of neuromyelitis optica in the acute phase.

Means to solve the problem

The present inventors have intensively studied for achieving the above object and found that the RGMa inhibiting substance, in particular the anti-RGMa neutralizing antibody has an effect of recovering from exacerbation of clinical symptom in acute phase neuromyelitis optica, an effect of quickly repairing the disruption of the blood spinal cord barrier caused by myelitis, and an effect of suppressing the granulocyte infiltration observed in the pathology of acute phase neuromyelitis optica, thereby the inhibiting substance exhibits an excellent effect on the prevention or treatment of acute phase neuromyelitis optica. They also found that a RGMa inhibiting substance, in particular an anti-RGMa neutralizing antibody is capable of treating, alleviating, or mitigating the pain symptoms in neuromyelitis optica, thereby completed the present invention.

The present invention is as follows.

1. An agent for preventing or treating acute phase neuromyelitis optica comprising a RGMa inhibiting substance.

2. The preventive or therapeutic agent according to item 1, wherein the RGMa inhibiting substance is an anti-RGMa neutralizing antibody.

3. The preventive or therapeutic agent according to item 2, wherein the anti-RGMa neutralizing antibody is a humanized antibody.

4. The preventive or therapeutic agent according to item 2 or 3, wherein the anti-RGMa neutralizing antibody is an antibody recognizing an amino acid sequence selected from SEQ ID NOS: 16, 36, 37, 38 and 39.

5. The preventive or therapeutic agent according to any one of item 2 to 4, wherein the anti-RGMa neutralizing antibody is an antibody selected from the following (a) to (1):

(a) an anti-RGMa neutralizing antibody comprising a light chain variable region comprising an LCDR1 comprising the amino acid sequence represented by SEQ ID NO: 5, an LCDR2 comprising the amino acid sequence represented by SEQ ID NO: 6, and an LCDR3 comprising the amino acid sequence represented by SEQ ID NO: 7, and a heavy chain variable region comprising an HCDR1 comprising the amino acid sequence represented by SEQ TD NO: 8, an HCDR2 comprising the amino acid sequence represented by SEQ TD NO: 9, and an HCDR3 comprising the amino acid sequence represented by SEQ ID NO: 10;

(b) an anti-RGMa neutralizing antibody comprising a light chain variable region comprising an LCDR1 comprising the amino acid sequence represented by SEQ ID NO: 11, an LCDR2 comprising the amino acid sequence represented by SEQ ID NO: 12, and an LCDR3 comprising the amino acid sequence represented by SEQ ID NO: 13, and a heavy chain variable region comprising an HCDR1 comprising the amino acid sequence represented by SEQ TD NO: 14, an HCDR2 comprising the amino acid sequence represented by SEQ ID NO: 15, and an HCDR3 comprising an amino acid sequence comprising SFG;

(c) an anti-RGMa neutralizing antibody comprising a light chain variable region comprising an LCDR1 comprising the amino acid sequence represented by SEQ ID NO: 17, an LCDR2 comprising the amino acid sequence represented by SEQ TD NO: 18, and an LCDR3 comprising the amino acid sequence represented by SEQ ID NO: 19, and a heavy chain variable region comprising an HCDR1 comprising the amino acid sequence represented by SEQ ID NO: 20, an HCDR2 comprising the amino acid sequence represented by SEQ ID NO: 21, and an HCDR3 comprising the amino acid sequence represented by SEQ ID NO: 22;

(d) an anti-RGMa neutralizing antibody comprising a light chain variable region comprising an LCDR1 comprising the amino acid sequence represented by SEQ ID NO: 23, an LCDR2 comprising the amino acid sequence represented by SEQ ID NO: 24, and an LCDR3 comprising the amino acid sequence represented by SEQ ID NO: 25, and a heavy chain variable region comprising an HCDR1 comprising the amino acid sequence represented by SEQ ID NO: 26, an HCDR2 comprising the amino acid sequence represented by SEQ TD NO: 27, and an HCDR3 comprising the amino acid sequence represented by SEQ ID NO: 28;

(e) an anti-RGMa neutralizing antibody comprising a light chain variable region comprising an LCDR1 comprising the amino acid sequence represented by SEQ ID NO: 29, an LCDR2 comprising the amino acid sequence represented by SEQ TD NO: 30, and an LCDR3 comprising the amino acid sequence represented by SEQ ID NO: 31, and a heavy chain variable region comprising an HCDR1 comprising the amino acid sequence represented by SEQ TD NO: 32, an HCDR2 comprising the amino acid sequence represented by SEQ TD NO: 33, and an HCDR3 comprising the amino acid sequence represented by SEQ ID NO: 34;

(f) an anti-RGMa neutralizing antibody comprising a light chain variable region comprising an LCDR1 comprising the amino acid sequence represented by SEQ ID NO: 29, an LCDR2 comprising the amino acid sequence represented by SEQ TD NO: 30, and an LCDR3 comprising the amino acid sequence represented by SEQ ID NO: 35, and a heavy chain variable region comprising an HCDR1 comprising the amino acid sequence represented by SEQ TD NO: 32, an HCDR2 comprising the amino acid sequence represented by SEQ TD NO: 33, and an HCDR3 comprising the amino acid sequence represented by SEQ ID NO: 34;

(g) an anti-RGMa neutralizing antibody comprising a light chain variable region comprising an LCDR1 comprising the amino acid sequence represented by SEQ ID NO: 29, an LCDR2 comprising the amino acid sequence represented by SEQ TD NO: 30, and an LCDR3 comprising the amino acid sequence represented by SEQ ID NO: 40, and a heavy chain variable region comprising an HCDR1 comprising the amino acid sequence represented by SEQ ID NO: 32, an HCDR2 comprising the amino acid sequence represented by SEQ ID NO: 33, and an HCDR3 comprising the amino acid sequence represented by SEQ ID NO: 34;

(h) an anti-RGMa neutralizing antibody comprising a light chain variable region comprising an LCDR1 comprising the amino acid sequence represented by SEQ ID NO: 29, an LCDR2 comprising the amino acid sequence represented by SEQ ID NO: 30, and an LCDR3 comprising the amino acid sequence represented by SEQ ID NO: 41, and a heavy chain variable region comprising an HCDR1 comprising the amino acid sequence represented by SEQ ID NO: 32, an HCDR2 comprising the amino acid sequence represented by SEQ TD NO: 33, and an HCDR3 comprising the amino acid sequence represented by SEQ ID NO: 34;

(i) an anti-RGMa neutralizing antibody comprising a light chain variable region comprising an LCDR1 comprising the amino acid sequence represented by SEQ ID NO: 29, an LCDR2 comprising the amino acid sequence represented by SEQ TD NO: 30, and an LCDR3 comprising the amino acid sequence represented by SEQ ID NO: 42, and a heavy chain variable region comprising an HCDR1 comprising the amino acid sequence represented by SEQ TD NO: 32, an HCDR2 comprising the amino acid sequence represented by SEQ TD NO: 33, and an HCDR3 comprising the amino acid sequence represented by SEQ ID NO: 34;

(j) an anti-RGMa neutralizing antibody comprising a light chain variable region comprising an LCDR1 comprising the amino acid sequence represented by SEQ ID NO: 29, an LCDR2 comprising the amino acid sequence represented by SEQ TD NO: 30, and an LCDR3 comprising the amino acid sequence represented by SEQ ID NO: 43, and a heavy chain variable region comprising an HCDR1 comprising the amino acid sequence represented by SEQ TD NO: 32, an HCDR2 comprising the amino acid sequence represented by SEQ TD NO: 33, and an HCDR3 comprising the amino acid sequence represented by SEQ ID NO: 34;

(k) an anti-RGMa neutralizing antibody comprising a light chain variable region comprising an LCDR1 comprising the amino acid sequence represented by SEQ ID NO: 29, an LCDR2 comprising the amino acid sequence represented by SEQ TD NO: 30, and an LCDR3 comprising the amino acid sequence represented by SEQ ID NO: 44, and a heavy chain variable region comprising an HCDR1 comprising the amino acid sequence represented by SEQ ID NO: 32, an HCDR2 comprising the amino acid sequence represented by SEQ ID NO: 33, and an HCDR3 comprising the amino acid sequence represented by SEQ ID NO: 34; and

(l) an anti-RGMa neutralizing antibody comprising a light chain variable region comprising an LCDR1 comprising the amino acid sequence represented by SEQ ID NO: 29, an LCDR2 comprising the amino acid sequence represented by SEQ ID NO: 30, and an LCDR3 comprising the amino acid sequence represented by SEQ ID NO: 45, and a heavy chain variable region comprising an HCDR1 comprising the amino acid sequence represented by SEQ ID NO: 32, an HCDR2 comprising the amino acid sequence represented by SEQ TD NO: 33, and an HCDR3 comprising the amino acid sequence represented by SEQ ID NO: 34.

6. An agent for preventing or treating pain symptoms in neuromyelitis optica, comprising a RGMa inhibiting substance.

7. The preventive or therapeutic agent according to item 6, wherein the RGMa inhibiting substance is an anti-RGMa neutralizing antibody.

8. The preventive or therapeutic agent according to item 7, wherein the anti-RGMa neutralizing antibody is a humanized antibody.

9. The preventive or therapeutic agent according to item 7 or 8, wherein the anti-RGMa neutralizing antibody is an antibody recognizing the amino acid sequence selected from SEQ ID NOS: 16, 36, 37, 38 and 39.

10. The preventive or therapeutic agent according to any one of items 7 to 9, wherein the anti-RGMa neutralizing antibody is an antibody selected from the following (a) to (1):

(a) an anti-RGMa neutralizing antibody comprising a light chain variable region comprising an LCDR1 comprising the amino acid sequence represented by SEQ ID NO: 5, an LCDR2 comprising the amino acid sequence represented by SEQ ID NO: 6, and an LCDR3 comprising the amino acid sequence represented by SEQ ID NO: 7, and a heavy chain variable region comprising an HCDR1 comprising the amino acid sequence represented by SEQ TD NO: 8, an HCDR2 comprising the amino acid sequence represented by SEQ ID NO: 9, and an HCDR3 comprising the amino acid sequence represented by SEQ ID NO: 10;

(b) an anti-RGMa neutralizing antibody comprising a light chain variable region comprising an LCDR1 comprising the amino acid sequence represented by SEQ ID NO: 11, an LCDR2 comprising the amino acid sequence represented by SEQ ID NO: 12, and an LCDR3 comprising the amino acid sequence represented by SEQ ID NO: 13, and a heavy chain variable region comprising an HCDR1 comprising the amino acid sequence represented by SEQ ID NO: 14, an HCDR2 comprising the amino acid sequence represented by SEQ ID NO: 15, and an HCDR3 comprising an amino acid sequence comprising SFG;

(c) an anti-RGMa neutralizing antibody comprising a light chain variable region comprising an LCDR1 comprising the amino acid sequence represented by SEQ ID NO: 17, an LCDR2 comprising the amino acid sequence represented by SEQ ID NO: 18, and an LCDR3 comprising the amino acid sequence represented by SEQ ID NO: 19, and a heavy chain variable region comprising an HCDR1 comprising the amino acid sequence represented by SEQ ID NO: 20, an HCDR2 comprising the amino acid sequence represented by SEQ ID NO: 21, and an HCDR3 comprising the amino acid sequence represented by SEQ ID NO: 22;

(d) an anti-RGMa neutralizing antibody comprising a light chain variable region comprising an LCDR1 comprising the amino acid sequence represented by SEQ ID NO: 23, an LCDR2 comprising the amino acid sequence represented by SEQ ID NO: 24, and an LCDR3 comprising the amino acid sequence represented by SEQ ID NO: 25, and a heavy chain variable region comprising an HCDR1 comprising the amino acid sequence represented by SEQ ID NO: 26, an HCDR2 comprising the amino acid sequence represented by SEQ ID NO: 27, and an HCDR3 comprising the amino acid sequence represented by SEQ ID NO: 28;

(e) an anti-RGMa neutralizing antibody comprising a light chain variable region comprising an LCDR1 comprising the amino acid sequence represented by SEQ ID NO: 29, an LCDR2 comprising the amino acid sequence represented by SEQ ID NO: 30, and an LCDR3 comprising the amino acid sequence represented by SEQ ID NO: 31, and a heavy chain variable region comprising an HCDR1 comprising the amino acid sequence represented by SEQ ID NO: 32, an HCDR2 comprising the amino acid sequence represented by SEQ ID NO: 33, and an HCDR3 comprising the amino acid sequence represented by SEQ ID NO: 34;

(f) an anti-RGMa neutralizing antibody comprising a light chain variable region comprising an LCDR1 comprising the amino acid sequence represented by SEQ ID NO: 29, an LCDR2 comprising the amino acid sequence represented by SEQ ID NO: 30, and an LCDR3 comprising the amino acid sequence represented by SEQ ID NO: 35, and a heavy chain variable region comprising an HCDR1 comprising the amino acid sequence represented by SEQ ID NO: 32, an HCDR2 comprising the amino acid sequence represented by SEQ ID NO: 33, and an HCDR3 comprising the amino acid sequence represented by SEQ ID NO: 34;

(g) an anti-RGMa neutralizing antibody comprising a light chain variable region comprising an LCDR1 comprising the amino acid sequence represented by SEQ ID NO: 29, an LCDR2 comprising the amino acid sequence represented by SEQ ID NO: 30, and an LCDR3 comprising the amino acid sequence represented by SEQ ID NO: 40, and a heavy chain variable region comprising an HCDR1 comprising the amino acid sequence represented by SEQ TD NO: 32, an HCDR2 comprising the amino acid sequence represented by SEQ ID NO: 33, and an HCDR3 comprising the amino acid sequence represented by SEQ ID NO: 34;

(h) an anti-RGMa neutralizing antibody comprising a light chain variable region comprising an LCDR1 comprising the amino acid sequence represented by SEQ ID NO: 29, an LCDR2 comprising the amino acid sequence represented by SEQ ID NO: 30, and an LCDR3 comprising the amino acid sequence represented by SEQ ID NO: 41, and a heavy chain variable region comprising an HCDR1 comprising the amino acid sequence represented by SEQ TD NO: 32, an HCDR2 comprising the amino acid sequence represented by SEQ ID NO: 33, and an HCDR3 comprising the amino acid sequence represented by SEQ ID NO: 34;

(i) an anti-RGMa neutralizing antibody comprising a light chain variable region comprising an LCDR1 comprising the amino acid sequence represented by SEQ ID NO: 29, an LCDR2 comprising the amino acid sequence represented by SEQ ID NO: 30, and an LCDR3 comprising the amino acid sequence represented by SEQ ID NO: 42, and a heavy chain variable region comprising an HCDR1 comprising the amino acid sequence represented by SEQ TD NO: 32, an HCDR2 comprising the amino acid sequence represented by SEQ ID NO: 33, and an HCDR3 comprising the amino acid sequence represented by SEQ ID NO: 34;

(j) an anti-RGMa neutralizing antibody comprising a light chain variable region comprising an LCDR1 comprising the amino acid sequence represented by SEQ ID NO: 29, an LCDR2 comprising the amino acid sequence represented by SEQ ID NO: 30, and an LCDR3 comprising the amino acid sequence represented by SEQ ID NO: 43, and a heavy chain variable region comprising an HCDR1 comprising the amino acid sequence represented by SEQ ID NO: 32, an HCDR2 comprising the amino acid sequence represented by SEQ ID NO: 33, and an HCDR3 comprising the amino acid sequence represented by SEQ ID NO: 34;

(k) an anti-RGMa neutralizing antibody comprising a light chain variable region comprising an LCDR1 comprising the amino acid sequence represented by SEQ ID NO: 29, an LCDR2 comprising the amino acid sequence represented by SEQ ID NO: 30, and an LCDR3 comprising the amino acid sequence represented by SEQ ID NO: 44, and a heavy chain variable region comprising an HCDR1 comprising the amino acid sequence represented by SEQ TD NO: 32, an HCDR2 comprising the amino acid sequence represented by SEQ ID NO: 33,

and an HCDR3 comprising the amino acid sequence represented by SEQ ID NO: 34; and

(I) an anti-RGMa neutralizing antibody comprising a light chain variable region comprising an LCDR1 comprising the amino acid sequence represented by SEQ ID NO: 29, an LCDR2 comprising the amino acid sequence represented by SEQ ID NO: 30, and an LCDR3 comprising the amino acid sequence represented by SEQ ID NO: 45, and a heavy chain variable region comprising an HCDR1 comprising the amino acid sequence represented by SEQ TD NO: 32, an HCDR2 comprising the amino acid sequence represented by SEQ ID NO: 33, and an HCDR3 comprising the amino acid sequence represented by SEQ ID NO: 34.

11. A method of preventing or treating acute phase neuromyelitis optica, or pain symptoms in neuromyelitis optica, which comprises administration of an effective dose of a RGMa inhibiting substance to a mammal in need of treatment.

12. The preventive or therapeutic method according to item 11, wherein the RGMa inhibiting substance is an anti-RGMa neutralizing antibody.

13. Use of a RGMa inhibiting substance in the manufacture of an agent for preventing or treating acute phase neuromyelitis optica.

ADVANTAGEOUS EFFECTS OF INVENTION

According to the present invention, a RGMa inhibiting substance, in particular, an anti-RGMa neutralizing antibody, for example, exhibits an effect of quickly repairing the disruption of the blood spinal cord barrier (blood brain barrier in the case of the brain), and therefore is useful as an agent for preventing or treating acute phase neuromyelitis optica.

Then, according to the present invention, the RGMa inhibiting substance, in particular, the anti-RGMa neutralizing antibody, for example, exhibits an effect of suppressing granulocyte infiltration to spinal cord observed in acute phase neuromyelitis optica, and therefore is useful as an agent for preventing or treating acute phase neuromyelitis optica.

Further, according to the present invention, a RGMa inhibiting substance, in particular, an anti-RGMa neutralizing antibody, can treat, alleviate or mitigate the pain symptoms seen in neuromyelitis optica, and is useful as an agent for preventing or treating such pain symptoms.

BRIEF DESCRIPTION OF THE DRAWINGS

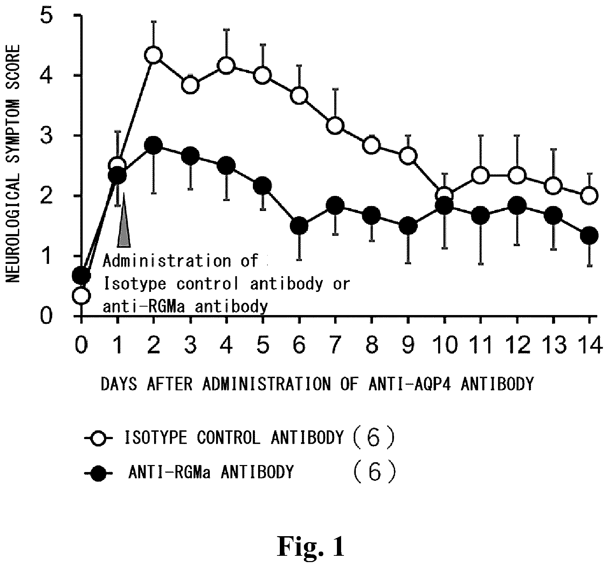

Fig. 1 is a graph showing the therapeutic effect of an anti-RGMa neutralizing antibody (may be simply referred to as anti-RGMa antibody) on exacerbation of clinical symptom in an acute phase of severe experimental NMO rat model. Each data represents a mean $\pm$ S.E.M. Each group size is shown in the parentheses in the graph legend.

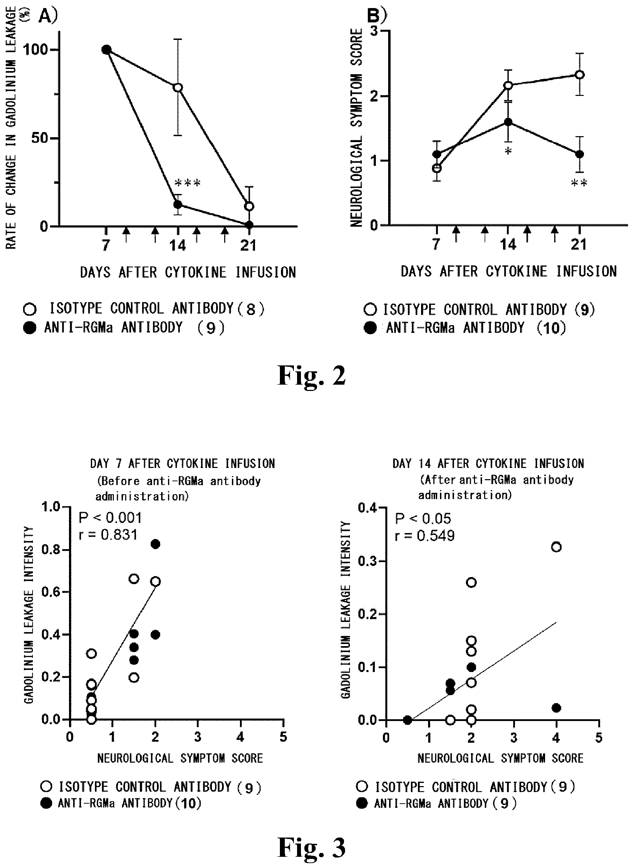

Fig. 2 is graphs showing the recovery effect of an anti-RGMa neutralizing antibody on the blood-spinal cord barrier disruption and neurological symptoms of tEAE mice in the acute phase. Panel A) in Fig.2 shows the recovery effect of an anti-RGMa neutralizing antibody on the alteration of gadolinium leakage level into spinal cord in the BSCB disruption in tEAE mice in the acute phase. Each data represents the mean $\pm$ S.E.M. Each group size is shown in the parentheses in the graph legend. The animals with no gadolinium leakage into spinal cord on 7 days after cytokine injection and unsuccessful MR image acquisition on 14 days after cytokine injection were excluded from analysis. Statistical analysis was performed using the Bonferroni multiple comparison test (***: $p < 0.001$). The arrows indicate the day of antibody administration. Panel B) in Fig.2 shows the recovery effect of an anti-RGMa neutralizing antibody on the neurological symptoms. Each data represents the mean $\pm$ S.E.M. of neurological symptom score and each group size is shown in the parentheses in the graph legend. The arrows indicate the day of antibody administration. The statistical analysis was performed at each time point using the Mann-Whitney U test (*: $p<0.05$, **: $p<0.01$).

Fig. 3 is graphs showing the correlation between the intensity of gadolinium intraspinal leakage and the severity of neurological symptoms in tEAE mice in the acute phase. The data of each individual is plotted and the Spearman's rank-correlation analysis was used for statistical analysis. Each group size is shown in the parentheses in the graph legend. There was one case in which MRI image-capturing was failed on day 14 of cytokine infusion (anti-RGMa neutralizing antibody-treated group).

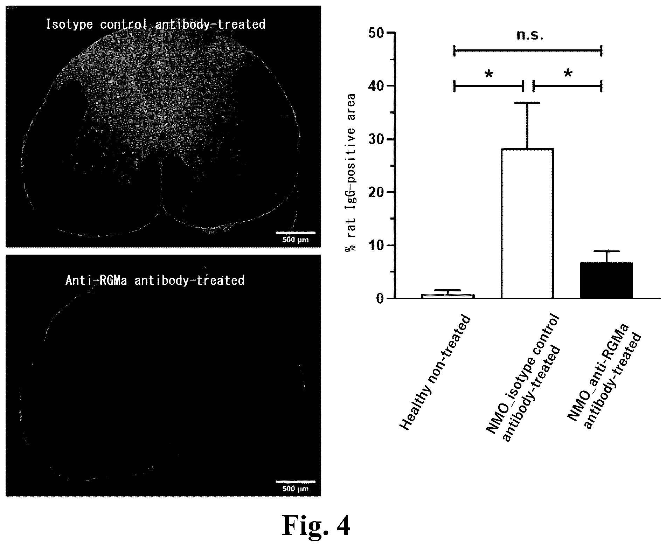

Fig. 4 is immunohistochemical images and a graph which show the therapeutic effect of the anti-RGMa neutralizing antibody on the blood-spinal cord barrier disruption in the acute phase of severe experimental NMO rat model. Each data in the quantitative analysis shows the mean $\pm$ S.E.M. value and was statistically analyzed with Tukey's multiple comparison test (*$p<0.05$).

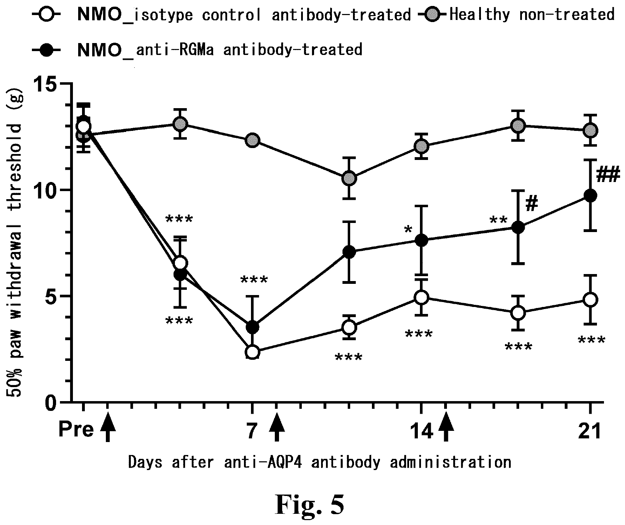

Fig. 5 is a graph which show the effect of the anti-RGMa neutralizing antibody on the pain-related behavior of the acute phase of severe experimental NMO rat model. Each data in the quantitative analysis shows the mean $\pm$

S.E.M. value and was statistically analyzed with Tukey's multiple comparison test (***p<0.001, **p<0.01, *p<0.05 vs healthy non-treated group, ## p<0.01, #p<0.05 vs NMO isotype control antibody-treated group). The arrow indicates the day of administration of the anti-RGMa neutralizing antibody or isotype control antibody.

Fig. 6 is immunohistochemical images and a graph which show the suppressing effect of the anti-RGMa neutralizing antibody on the granulocyte infiltration in the acute phase of severe experimental NMO rat model. Each data in the quantitative analysis shows the mean ± S.E.M. value and was statistically analyzed with Tukey's multiple comparison test (**p<0.01, *p<0.05).

Fig. 7 is immunohistochemical images which show the expression of RGMa at the AQP4-loss sites in the spinal cord of the acute phase of severe experimental NMO rat model.

DETAILED DESCRIPTION OF THE INVENTION

The terms used in the present invention will be described below.

[Neutralization]

The term "neutralization" as used herein refers to an action of binding to a target of interest and inhibiting functions of the target. For example, a RGMa inhibiting substance refers to a substance which binds to RGMa and consequently acts to inhibit a biological activity of RGMa.

[Epitope]

As used herein, the term "epitope" includes a polypeptide determinant that can specifically bind to an immunoglobulin or a T-cell receptor. In some embodiments, an epitope can include a chemically active group on the surface of the molecule (for example, an amino acid, a sugar side chain, phosphoryl or sulfonyl). Tn some embodiments, an epitope can have particular characteristics of three-dimensional structure and/or electric charge. Epitopes refer to regions in antigens, to which antibodies bind.

[Isolated]

As used herein, the term "isolated" such as in isolated RGMa inhibiting substance (for example, antibody) means being identified, and separated and/or recovered from components in natural states. Impurities in natural states are substances that can interfere with the diagnostic or therapeutic use of the antibody, including enzymes, hormones and other proteinous or nonproteinous solutes. Generally, RGMa inhibiting substances or the like may be isolated through at least one purification step. RGMa inhibiting substances purified through at least one purification step can be referred to as "isolated RGMa inhibiting substances".

[Antibody]

As used herein, the term "antibody" refers broadly to an immunoglobulin (Ig) molecule comprising four polypeptide chains, namely two heavy chains (H chains) and two light chains (L chains), that substantially retain the characteristics of an Ig molecule to bind to an epitope.

[Human Antibody]

As used herein, the term "human antibody" refers to an antibody comprising light and heavy chains which are both derived from human immunoglobulins. Depending on the difference in the constant region of the heavy chain, human antibodies include IgG comprising a $\gamma$ heavy chain (including IgG1, IgG2, IgG3 and IgG4); IgM comprising a $\mu$ heavy chain; IgA comprising an $\alpha$ heavy chain (including IgA1 and TgA2); IgD comprising a $\delta$ heavy chain; and IgE comprising an $\varepsilon$ heavy chain. In principle, a light chain comprises either $\kappa$ or $\lambda$ chain.

[Humanized Antibody]

As used herein, the term "humanized antibody" refers to an antibody comprising variable regions comprising complementarity determining regions from antibodies derived from non-human animals and framework regions derived from human antibodies, and constant regions derived from human antibodies.

[Chimeric Antibody]

As used herein, the term "chimeric antibody" refers to an antibody in which the light chain, the heavy chain, or both comprise a non-human derived variable region and a human derived constant region.

[Monospecific Antibody]

As used herein, the term "monospecific antibody" refers to an antibody comprising a single independent antigen recognition site having a single antigen specificity. For example, a monospecific antibody that recognizes RGMa may be referred herein to as a RGMa-monospecific antibody.

[Multispecific Antibody]

As used herein, the term "multispecific antibody" refers to antibodies comprising two or more independent antigen recognition sites having two or more different antigen specificities, including bispecific antibodies having two antigen specificities and trispecific antibodies having three antigen specificities.

[Complementarity Determining Region (CDR)]

The term "complementarity determining region (CDR)" refers to a region forming an antigen binding site in a variable region of an immunoglobulin molecule, which is also called a hypervariable region, and particularly refers to a portion in which the amino acid sequence changes greatly for each immunoglobulin molecule. As CDRs, light and heavy chains each have three CDRs. Three CDRs contained in a light chain may be referred to as LCDR1, LCDR2, and LCDR3, while three CDRs contained in a heavy chain may be referred to as HCDR1, HCDR2, and HCDR3. For example, CDRs of an immunoglobulin molecule are assigned according to the Kabat numbering system (Kabat, et al., 1987, Sequences of Proteins of Immunological Interest, US Department of Health and Human Services, NIH, USA).

[Effective Dose]

The term "effective dose" refers to a sufficient dose of a preventive or therapeutic agent to alleviate or ameliorate the severity and/or duration of a disorder, or one or more symptoms thereof; to prevent progression of the disorder; to reverse the disorder; to prevent the recurrence, onset, development, or progression of one or more symptoms associated with the disorder; to detect the disorder; or to enhance or improve one or more preventive or therapeutic effects of another therapy (for example, a prophylactic drug or a therapeutic drug).

[Percent (%) Identity of Amino Acid Sequence]

"Percent (%) identity" of the amino acid sequence of a candidate polypeptide sequence, such as a variable region, with respect to the amino acid sequence of a reference polypeptide sequence is defined as a percent of the same amino acid residues in the candidate sequence as the amino acid residues in the particular reference polypeptide sequence, which percent is obtained by arranging the sequences and introducing gaps if necessary to obtain maximal % identity, without considering any conservative substitutions as part of the sequence identity. Alignment for determination of % identity can be accomplished by using various methods within the skill of the art, for example, using a publicly available computer software, such as BLAST, BLAST-2, ALIGN, or Megalign (DNASTAR) software. Those skilled in the art can determine appropriate parameters for sequence alignment, including algorithm needed to achieve maximal alignment over the full length of the sequence to be compared. However, for the purposes herein, values of % identity are obtained in pairwise alignment using a computer program for comparing sequences, BLAST.
When BLAST is used in comparison of amino acid sequences, the % identity of a given amino acid sequence A to a given amino acid sequence B is calculated as follows:

a fraction X/Y multiplied by 100
where X is the number of amino acid residues scored as identical in a programmed alignment of A and B by the sequence alignment program Blast, and Y is the total number of amino acid residues in B. It will be understood that when the lengths of amino acid sequences A and B are different, the % identities of A to B and of B to A are different. Unless stated otherwise, all % identity values herein are obtained using a computer program BLAST, as described in the immediately preceding paragraph.

[Conservative Substitution]

"Conservative substitution" means replacement of an amino acid residue with another chemically similar amino acid residue so as not to substantially alter the activity of the peptide. Examples thereof include a case where a hydrophobic residue is substituted with another hydrophobic residue, and a case where a polar residue is substituted with another polar residue having the same charge. Examples of functionally similar amino acids eligible for such substitution include, as for non-polar (hydrophobic) amino acids, alanine, valine, isoleucine, leucine, proline, tryptophan, phenylalanine, and methionine. As for polar (neutral) amino acids, they include glycine, serine, threonine, tyrosine, glutamine, asparagine, and cysteine. As for positively charged (basic) amino acids, they include arginine, histidine, and lysine. As for negatively charged (acidic) amino acids, they include aspartic acid, and glutamic acid.

The present invention will be described in detail below.

The present invention provides an agent for preventing or treating acute phase neuromyelitis optica, which is a novel use of a RGMa inhibiting substance.

Further, the present invention provides a method of preventing or treating acute phase neuromyelitis optica, comprising a step of administering an agent for preventing or treating acute phase neuromyelitis optica comprising an effective dose of a RGMa inhibiting substance to a mammal in need of treatment.

<RGMa inhibiting substance>

There is no particular restriction on the RGMa inhibiting substance of the present invention, insofar as it is a substance that acts on RGMa itself to inhibit or reduce the activity of RGMa (hereinafter sometimes referred to simply as "RGMa activity"). For example, a substance having an activity that directly inhibits (or reduces) the RGMa activity by binding to the RGMa, or an activity that indirectly inhibits (or reduces) the RGMa activity by inhibiting the binding of a RGMa to a receptor (e.g., the compound, antibody, etc. described below) is referred to as the RGMa inhibiting substance of the present invention.

The RGMa inhibiting substance of this invention may also be a substance that inhibits the expression of RGMa, for example, a substance that inhibits the expression of RGMa to inhibit (reduce) the RGMa activity (e.g., a nucleic acid molecule described below) is also included in the RGMa inhibiting substance of the present invention.

RGMa is identified as a protein that inhibits neurite outgrowth in the central nervous system. A human RGMa protein is biosynthesized as a precursor protein comprising 450 amino acids as shown in SEQ ID NO: 1. The signal peptide from Met 1 to Pro 47 present at the N terminus (which refers to the peptide from the methionine residue at position 1 to the proline residue at position 47 from the N-terminal side, and is hereafter described as above) is removed. Then, the peptide bond between Asp 168 and Pro 169 is cleaved to generate an N-terminal domain. In the C-terminal fragment from Pro 169, the peptide from Ala 425 to Cys 450 at the C terminus is removed so that Ala 424 becomes the C terminus. Then, a GPI anchor is added to the C-terminal carboxyl group of Ala 424 to generate a C-terminal domain. The human RGMa protein is expressed on the cell membrane via a GPI anchor as a mature protein in which the N-terminal domain (Cys 48 to Asp 168) and the C-terminal domain (Pro 169 to Ala 424) are linked by a disulfide bond.

RGMa in the present invention may be derived from any animals. Preferably, RGMa is human RGMa. A human RGMa precursor protein comprises the amino acid sequence shown in SEQ ID NO: 1 in Sequence Listing. A mouse RGMa precursor protein comprises the amino acid sequence shown in SEQ ID NO: 2 in Sequence Listing, while a rat RGMa precursor protein comprises the amino acid sequence shown in SEQ ID NO: 3 in Sequence Listing. Removal of the C-terminal peptides from each amino acid sequence results in mature proteins having the same amino acid sequence, respectively.

RGMa genes include, but are not limited to, a human RGMa gene comprising the nucleotide sequence shown in SEQ ID NO: 4. Nucleotide sequences of RGM genes derived from various organisms can be easily obtained from known databases (for example, GenBank).

Specific examples of the RGMa inhibiting substance of the present invention include low molecular weight compounds, anti-RGMa neutralizing antibodies, and functionally modified antibodies thereof, conjugated antibodies thereof, and antigen-binding fragments thereof, as well as an siRNA (short interfering RNA), an shRNA (short hairpin RNA), and an antisense oligonucleotide, which are nucleic acid molecules against RGMa. Among these RGMa inhibiting substances, an anti-RGMa neutralizing antibody, a functionally modified antibody thereof, a conjugated antibody thereof, and an antigen-binding fragment thereof are preferable, an anti-RGMa neutralizing antibody, and an antigen-binding fragment thereof are more preferable, and an anti-RGMa neutralizing antibody is especially preferable.

<Anti-RGMa Neutralizing Antibody>

According to the present invention, the anti-RGMa neutralizing antibody is an antibody that binds to RGMa to neutralize the RGMa activity, and may be a polyclonal, or monoclonal antibody. A monoclonal antibody is more preferable according to the present invention. The anti-RGMa-neutralizing antibody of the present invention may be a RGMa-monospecific antibody, or a multispecific antibody that recognizes RGMa and a plurality of other antibodies. A RGMa-monospecific antibody is more preferable.

Specifically, the epitopes in human RGMa are preferably one or more of SEQ ID NO: 16 (amino acid numbers 47 to 69 in SEQ ID NO: 1), SEQ ID NO: 36 (amino acid numbers 298 to 311 in SEQ ID NO: 1), SEQ ID NO: 37 (amino acid numbers 322 to 335 in SEQ ID NO: 1), SEQ ID NO: 38 (amino acid numbers 349 to 359 in SEQ ID NO: 1), and SEQ ID NO: 39 (amino acid numbers 367 to 377 in SEQ ID NO: 1); more preferably a combination of SEQ ID NOS: 36 and 37; and particularly preferably a combination of SEQ ID NOS: 36, 37 and 39.

The anti-RGMa neutralizing antibodies of the present invention include polyclonal or monoclonal antibodies obtained by immunizing mammals such as mice with an antigen which is a RGMa protein or a partial fragment thereof (for example, epitope fragment described above); chimeric antibodies and humanized antibodies produced by gene recombination technology; and human antibodies produced, for example, by a transgenic animal producing human antibody. When the antibody of the present invention is administered to a human as a medicine, the antibody is desirably a humanized antibody or a human antibody from the viewpoint of reducing side effects.

Specific examples of the anti-RGMa neutralizing antibody of the present invention include the following antibodies (a) to (1). As the respective production methods therefor, the methods described in Patent Documents 2 to 4 may be used.

Examples thereof include antibodies to be selected from:

(a) an anti-RGMa neutralizing antibody comprising a light chain variable region comprising an LCDR1 comprising the amino acid sequence represented by SEQ ID NO: 5, an LCDR2 comprising the amino acid sequence represented by SEQ TD NO: 6 and an LCDR3 comprising the amino acid sequence represented by SEQ ID NO: 7, and a heavy chain variable region comprising an HCDR1 comprising the amino acid sequence represented by SEQ ID NO: 8, an HCDR2 comprising the amino acid sequence represented by SEQ ID NO: 9 and an HCDR3 comprising the amino acid sequence represented by SEQ ID NO: 10 (the anti-RGMa neutralizing antibody also includes antibodies whose epitopes are the amino acid sequences represented by SEQ ID NOS: 36, 37 and 39);

(b) an anti-RGMa neutralizing antibody comprising a light chain variable region comprising an LCDR1 comprising the amino acid sequence represented by SEQ ID NO: 11, an LCDR2 comprising the amino acid sequence represented by SEQ ID NO: 12 and an LCDR3 comprising the amino acid sequence represented by SEQ ID NO: 13, and a heavy chain variable region comprising an HCDR1 comprising the amino acid sequence represented by SEQ ID NO: 14, an HCDR2 comprising the amino acid sequence represented by SEQ ID NO: 15 and an HCDR3 comprising an amino acid sequence comprising SFG (the anti-RGMa neutralizing antibody also includes antibodies whose epitopes are the amino acid sequences represented by SEQ ID NOS: 36, 37 and 38);

(c) an anti-RGMa neutralizing antibody comprising a light chain variable region comprising an LCDR1 comprising the amino acid sequence represented by SEQ ID NO: 17, an LCDR2 comprising the amino acid sequence represented by SEQ ID NO: 18 and an LCDR3 comprising the amino acid sequence represented by SEQ TD NO: 19, and a heavy chain variable region comprising an HCDR1 comprising the amino acid sequence represented by SEQ ID NO: 20, an HCDR2 comprising the amino acid sequence represented by SEQ ID NO: 21 and an HCDR3 comprising the amino acid sequence represented by SEQ ID NO: 22;

(d) an anti-RGMa neutralizing antibody comprising a light chain variable region comprising an LCDR1 comprising the amino acid sequence represented by SEQ ID NO: 23, an LCDR2 comprising the amino acid sequence represented by SEQ ID NO: 24 and an LCDR3 comprising the amino acid sequence represented by SEQ TD NO: 25, and a heavy chain variable region comprising an HCDR1 comprising the amino acid sequence represented by SEQ ID NO: 26, an HCDR2 comprising the amino acid sequence represented by SEQ ID NO: 27 and an HCDR3 comprising the amino acid sequence represented by SEQ ID NO: 28;

(e) an anti-RGMa neutralizing antibody comprising a light chain variable region comprising an LCDR1 comprising the amino acid sequence represented by SEQ ID NO: 29, an LCDR2 comprising the amino acid sequence represented by SEQ ID NO: 30 and an LCDR3 comprising the amino acid sequence represented by SEQ ID NO: 31, and a heavy chain variable region comprising an HCDR1 comprising the amino acid sequence represented by SEQ TD NO: 32, an HCDR2 comprising the amino acid sequence represented by SEQ ID NO: 33 and an HCDR3 comprising the amino acid sequence represented by SEQ ID NO: 34 (the anti-RGMa neutralizing antibody also includes an antibody whose epitope is the amino acid sequence represented by SEQ ID NO: 16);

(f) an anti-RGMa neutralizing antibody comprising a light chain variable region comprising an LCDR1 comprising the amino acid sequence represented by SEQ ID NO: 29, an LCDR2 comprising the amino acid sequence represented by SEQ ID NO: 30 and an LCDR3 comprising the amino acid sequence represented by SEQ ID NO: 35, and a heavy chain variable region comprising an HCDR1 comprising the amino acid sequence represented by SEQ TD NO: 32, an HCDR2 comprising the amino acid sequence represented by SEQ TD NO: 33 and an HCDR3 comprising the amino acid sequence represented by SEQ ID NO: 34 (the anti-RGMa neutralizing antibody also includes an antibody whose epitope is the amino acid sequence represented by SEQ ID NO: 16);

(g) an anti-RGMa neutralizing antibody comprising a light chain variable region comprising an LCDR1 comprising the amino acid sequence represented by SEQ ID NO: 29, an LCDR2 comprising the amino acid sequence represented by SEQ ID NO: 30 and an LCDR3 comprising the amino acid sequence represented by SEQ ID NO: 40, and a heavy chain variable region comprising an HCDR1 comprising the amino acid sequence represented by SEQ TD NO: 32, an HCDR2 comprising the amino acid sequence represented by SEQ ID NO: 33 and an HCDR3 comprising the amino acid sequence represented by SEQ ID NO: 34 (the anti-RGMa neutralizing antibody also includes an antibody whose epitope is the amino acid sequence represented by SEQ ID NO: 16);

(h) an anti-RGMa neutralizing antibody comprising a light chain variable region comprising an LCDR1 comprising the amino acid sequence represented by SEQ ID NO: 29, an LCDR2 comprising the amino acid sequence represented by SEQ ID NO: 30 and an LCDR3 comprising the amino acid sequence represented by SEQ ID NO: 41, and a heavy chain variable region comprising an HCDR1 comprising the amino acid sequence represented by SEQ ID NO: 32, an HCDR2 comprising the amino acid sequence represented by SEQ TD NO: 33 and an HCDR3 comprising the amino acid sequence represented by SEQ ID NO: 34 (the anti-RGMa neutralizing antibody also includes an antibody whose epitope is the amino acid sequence represented by SEQ ID NO: 16);

(i) an anti-RGMa neutralizing antibody comprising a light chain variable region comprising an LCDR1 comprising the amino acid sequence represented by SEQ ID NO: 29, an LCDR2 comprising the amino acid sequence represented by SEQ ID NO: 30 and an LCDR3 comprising the amino acid sequence represented by SEQ ID NO: 42, and a heavy chain variable region comprising an HCDR1 comprising the amino acid sequence represented by SEQ ID NO: 32, an HCDR2 comprising the amino acid sequence represented by SEQ ID NO: 33 and an HCDR3 comprising the amino acid sequence represented by SEQ ID NO: 34 (the anti-RGMa neutralizing antibody also includes an antibody whose epitope is the amino acid sequence represented by SEQ ID NO: 16);

(j) an anti-RGMa neutralizing antibody comprising a light chain variable region comprising an LCDR1 comprising the amino acid sequence represented by SEQ ID NO: 29, an LCDR2 comprising the amino acid sequence represented by SEQ ID NO: 30 and an LCDR3 comprising the amino acid sequence represented by SEQ ID NO: 43, and a heavy chain variable region comprising an HCDR1 comprising the amino acid sequence represented by SEQ TD NO: 32, an HCDR2 comprising the amino acid sequence represented by SEQ TD NO: 33 and an HCDR3 comprising the amino acid sequence represented by SEQ ID NO: 34 (the anti-RGMa neutralizing antibody also includes an antibody whose epitope is the amino acid sequence represented by SEQ ID NO: 16);

(k) an anti-RGMa neutralizing antibody comprising a light chain variable region comprising an LCDR1 comprising the amino acid sequence represented by SEQ ID NO: 29, an LCDR2 comprising the amino acid sequence represented by SEQ ID NO: 30 and an LCDR3 comprising the amino acid sequence represented by SEQ ID NO: 44, and a heavy chain variable region comprising an HCDR1 comprising the amino acid sequence represented by SEQ TD NO: 32, an HCDR2 comprising the amino acid sequence represented by SEQ ID NO: 33 and an HCDR3 comprising the amino acid sequence represented by SEQ ID NO: 34 (the anti-RGMa neutralizing antibody also includes an antibody whose epitope is the amino acid sequence represented by SEQ ID NO: 16); and

(l) an anti-RGMa neutralizing antibody comprising a light chain variable region comprising an LCDR1 comprising the amino acid sequence represented by SEQ ID NO: 29, an LCDR2 comprising the amino acid sequence represented by SEQ ID NO: 30 and an LCDR3 comprising the amino acid sequence represented by SEQ ID NO: 45, and a heavy chain variable region comprising an HCDR1 comprising the amino acid sequence represented by SEQ ID NO: 32, an HCDR2 comprising the amino acid sequence represented by SEQ TD NO: 33 and an HCDR3 comprising the amino acid sequence represented by SEQ ID NO: 34 (the anti-RGMa neutralizing antibody also includes an antibody whose epitope is the amino acid sequence represented by SEQ ID NO: 16).

Among these the antibody according to (a) is particularly preferable.

The anti-RGMa neutralizing antibodies of the present invention can be produced using production methods that are conventionally and commonly used. Antigens may be directly used for immunization, or may be used as a complex with a carrier protein. For preparing a complex of an antigen and a carrier protein, condensing agents such as glutaraldehyde, carbodiimides, and maleimide active esters can be used. Examples of the carrier protein include bovine serum albumin, thyroglobulin, hemocyanin, and KLH.

Examples of the mammal to be immunized include mice, rats, hamsters, guinea pigs, rabbits, cats, dogs, pigs, goats, horses and cattle. Examples of the inoculation method include subcutaneous, intramuscular and intraperitoneal administrations. When administered, antigens may be administered in mixture with complete or incomplete Freund's adjuvant, and are usually administered once every 2 to 5 weeks. Antibody-producing cells obtained from the spleen or lymph nodes of the immunized animals are fused with myeloma cells and isolated as hybridomas. As the myeloma cells, those derived from mammals such as mouse, rat, and human are used.

<Polyclonal Antibody>

The polyclonal antibodies can be obtained, for example, from a serum obtained from an immunized animal which is the mammal as described above immunized with the antigen as described above, if necessary, in combination with a Freund's adjuvant.

<Monoclonal Antibody>

Specifically, the monoclonal antibodies can be obtained, for example, as follows. That is, the antigen as described above is used as an immunogen, and the immunogen is injected or transplanted once or several times in combination with a Freund's adjuvant, as necessary, to the mammal as described above subcutaneously, intramuscularly, intravenously, into a footpad, or intraperitoneally for immunization. Typically, the immunization is performed about once to four times every 1 day to 14 days from the initial immunization, and after about 1 day to 5 days from the final immunization, antibody-producing cells are obtained from the immunized mammal.

The monoclonal antibodies can be obtained by a method well known to a person skilled in the art (for example, "Current Protocols in Molecular Biology" (John Wiley & Sons (1987)), or Antibodies: A Laboratory Manual, Ed. Harlow and David Lane, Cold Spring Harbor Laboratory (1988)).

The "hybridomas" secreting monoclonal antibodies can be prepared according to the method of Köhler and Milstein, et al. (Nature, 256, 495, 1975) or a modified method based on it. That is, they are prepared by cell fusion between an antibody-producing cell included in the spleen etc. obtained from an immunized mammal, and a myeloma cell, which is not capable of producing an autoantibody, and derived from a mammal, preferably mouse, rat or human.

Examples of the myeloma cell which can be used in the cell fusion include mouse-derived myelomas such as P3/X63-AG8.653 (653), P3/NST/1-Ag4-1 (NS-1), P3/X63-Ag8.U1 (P3U1), SP2/0-Agl4 (Sp2/O, Sp2), PAI, F0 and BW5147; rat-derived myelomas such as 210RCY3-Ag.2.3.; and human-derived myelomas such as U-266AR1, GM1500-6TG-A1-2, UC729-6, CEM-AGR, D1R11 and CEM-T15.

Examples of the fusion promoting agent include polyethylene glycols. The cell fusion can usually be performed by a reaction in a temperature range from 20°C to 40°C, preferably from 30°C to 37°C, for about 1 minute to 10 minutes usually using a polyethylene glycol (with an average molecular weight from 1000 to 4000) with a concentration from about 20% to 50%, wherein the ratio of the number of antibody-producing cells to the number of myeloma cells is usually from about 1:1 to 10:1

Screening of hybridoma clones producing the monoclonal antibodies can be carried out by culturing the hybridomas, for example, in a microtiter plate and assaying the culture supernatants in the wells for the reactivity against an immunogen by an immunochemical method such as ELISA.

In the screening of antibody-producing hybridomas, whether the antibody inhibits the RGMa activity in the present invention is also determined in addition to the binding assay with a RGMa protein. The screening methods allow for selection of the anti-RGMa neutralizing antibody of the present invention.

Clones can be further obtained from the wells containing hybridomas producing the desired antibodies by cloning with limiting dilution. Hybridomas are usually selected and grown in an animal cell culture medium containing 10% to 20% fetal bovine serum, supplemented with HAT (hypoxanthine, aminopterin and thymidine).

The monoclonal antibodies can be produced from the hybridomas by culturing the hybridomas *in vitro,* or growing them *in vivo,* for example, in ascitic fluids of mammals such as mice and rats, and isolating the monoclonal antibodies from the resulting culture supernatants or the ascitic fluids of the mammals.

When cultured *in vitro,* it is possible to use a nutrient medium suitable for growing, maintaining, and conserving hybridomas corresponding to various conditions such as the characteristics of the cell species to be cultured, or the culturing method, and producing a monoclonal antibody in the culture supernatant. Examples of the nutrient medium may include a known nutrient medium, or a nutrient medium prepared from a basal medium.

Examples of the basal medium include low-calcium media such as Ham's F12 medium, MCDB 153 medium, and low-calcium MEM medium, and high-calcium media such as MCDB 104 medium, MEM medium, D-MEM medium, RPMI 1640 medium, ASF 104 medium, and RD medium. The basal medium can contain, for example, sera, hormones, cytokines and/or various inorganic or organic substances according to the purpose.

The monoclonal antibodies can be isolated and purified by, for example, subjecting the above-described culture supernatant or ascitic fluid to saturated ammonium sulfate, euglobulin precipitation, a caproic acid treatment, a caprylic acid treatment, an ion exchange chromatography (such as DEAE or DE52), or an affinity column chromatography, for example with an anti-immunoglobulin column or a protein A column. Specifically, the monoclonal antibodies may be purified by any method known as an immunoglobulin purification method, and the purification can be easily achieved by means such as ammonium sulfate fractionation, PEG fractionation, ethanol fractionation, a method utilizing an anion exchanger, and further an affinity chromatography using a RGMa protein.

The monoclonal antibodies can also be obtained by a phage display method. In a phage display method, phages selected from an optional phage antibody library are screened using a desired immunogen, and phages having a desired binding ability to the immunogen are selected. Next, the antibody-corresponding sequence contained in the phage is isolated or sequenced. Based on the information of the isolated sequence or the determined sequence by the sequencing, an expression vector comprising a nucleic acid molecule encoding the antibody or antigen binding domain is constructed. The expression vector is then transfected into a cell line, and the cell line can be cultured to produce a monoclonal antibody. When a human antibody library is used as the phage antibody library, a human antibody having a desired binding ability can be produced.

<Nucleic Acid Molecule>

A nucleic acid molecule encoding an anti-RGMa neutralizing antibody of the present invention or an antigen-binding fragment thereof can be obtained, for example, by the following method. First, total RNA is prepared from a cell such as hybridoma using a commercially available RNA extraction kit, and subsequently cDNAs are synthesized with a reverse transcriptase using random primers and the like. Next, by a PCR method using, as primers, oligonucleotides of sequences respectively conserved in the variable regions in the known human antibody heavy chain and light chain genes, cDNAs encoding the antibody are amplified. Sequences encoding the constant regions can be obtained by amplification of known sequences by a PCR method. The nucleotide sequence of the DNA can be sequenced by a conventional method, for example, by incorporating it into a plasmid for sequencing.

Alternatively, a DNA encoding the monoclonal antibody of the present invention can also be obtained by chemically synthesizing sequences of the variable regions or parts thereof and joining them to sequences comprising the constant regions.

The nucleic acid molecule may encode all of the constant regions and the variable regions of heavy and light chains, or may encode only the variable regions of heavy and light chains. In the case of encoding all of the constant regions and the variable regions, the nucleotide sequences of the constant regions of heavy and light chains are preferably those described in Nucleic Acids Research, vol. 14, p1779, 1986; The Journal of Biological Chemistry, vol. 257, p1516, 1982; or Cell, vol. 22, p197, 1980.

<Functionally Modified Antibody>

A functionally modified antibody of an anti-RGMa neutralizing antibody is prepared by the following method. For example, when an anti-RGMa neutralizing antibody of this invention is produced using CHO cells as host cells, in which the $\alpha$1,6-fucosyl transferase (FUT8) gene is destructed, the fucose content in the sugar chain is decreased, and an antibody with an increased cell killing function is obtained. Meanwhile, when the same is produced using CHO cells as host cells, in which the FUT8 gene is transduced an antibody with a weak cell killing function is obtained (International Publication Nos. WO2005/035586, WO2002/31140, andWO00/61739). Meanwhile, the complement activation function can be modulated by modifying the amino acid residues in the Fc region (U.S. Patent Nos. 6,737,056, 7,297,775, and 7,317,091). Further, when the variant of the Fc region with enhanced binding to FcRn, which is one of Fc receptors, is used, the half-life in blood can be prolonged (Hashiguchi Shuhei, et al, Seikagaku, 2010, Vol. 82 (8), p710). These functionally modified antibodies can be produced by genetic engineering.

<Conjugated Antibody>

Examples of a modified molecule of the anti-RGMa neutralizing antibody of the present invention include a conjugated antibody. Examples of the conjugated antibody include a conjugated antibody in which an anti-RGMa neutralizing antibody is bound chemically or by a genetic engineering technique to a functional molecule other than the present anti-RGMa neutralizing antibody such as nonpeptidic polymers such as polyethylene glycol (PEG), radioactive substances, toxins, low molecular weight compounds, cytokines, growth factors (such as TGF-$\beta$, NGF, and neurotrophin), albumins, enzymes, and other antibodies.

When PEG is bound as the functional molecule, PEG having, but without limitation, a molecular weight from 2,000 Da to 100,000 Da, more preferably from 10,000 Da to 50,000 Da can be used. PEG may be linear or branched. PEG can be bound to the N-terminal amino group in the amino acids of an anti-RGMa neutralizing antibody, etc., for example by using an NHS active group.

When a radioactive substance is used as the functional molecule, its examples include $^{131}$I , $^{125}$I, $^{90}$Y, $^{64}$Cu, $^{99}$Tc, $^{77}$Lu, and $^{211}$At. The radioactive substance can be directly bound to the anti-RGMa neutralizing antibody by a chloramine-T method or the like.

When a toxin is used as the functional molecule, examples thereof include bacterial toxins (such as diphtheria toxin), phytotoxins (such as ricin), low molecular weight toxins (such as geldanamycin), maytansinoids and calicheamicins.

When a low molecular weight compound is used as the functional molecule, examples include daunomycin, doxorubicin, methotrexate, mitomycin, neocarzinostatin, vindesine, and fluorescent dyes such as FITC.

When an enzyme is used as the functional molecule, examples include luciferases (such as firefly luciferases, and bacterial luciferases; US Patent No. 4,737,456), malate dehydrogenases, ureases, peroxidases (such as horseradish peroxidase (HRPO)), alkaline phosphatases, $\beta$-galactosidases, glucoamylases, lysozymes, saccharide oxidases (such as glucose oxidase, galactose oxidase, and glucose-6-phosphate dehydrogenase), heterocyclic oxidases (such as uricase, and xanthine oxidase), lactoperoxidases, and microperoxidases.

Examples of linkers used for chemically bonding the toxin, low molecular weight compound, or enzyme include divalent radicals (such as alkylene, arylene, and heteroarylene), linkers represented by $-(CR_2)_nO(CR_2)_n-$ (wherein R is any substituent, and n is a positive integer), alkoxy repeating units (such as polyethyleneoxy, PEG, and polymethyleneoxy), alkylamino (such as polyethyleneamino, and Jeffamine(trademark)), and diacid esters and amides (including succinate, succinamide, diglycolate, malonate, and caproamide). Chemical modification methods for binding functional molecules have already been established in this field (D. J. King., Applications and Engineering of Monoclonal Antibodies., 1998, T.J. International Ltd, Monoclonal Antibody-Based Therapy of Cancer., 1998, Marcel Dekker Tnc.; Chari, et al., Cancer Res., 1992 Vol. 152: 127; and Liu, et al., Proc Natl Acad Sci USA., 1996 Vol 93: 8681).

<Antigen-binding Fragment>

In embodiments of the present invention, "antigen-binding fragments" of antibodies means partial regions having antigen binding properties derived from the antibodies as described above. Specific examples of the fragments include F(ab')$_2$, Fab', Fab, Fv (variable fragment of antibody), disulfide-linked Fv, single-chain antibodies (scFv), and polymers thereof. Examples of the antigen-binding fragments also include conjugated fragments that bind, chemically or by genetic engineering, functional molecules other than the present anti-RGMa neutralizing antibody, such as nonpeptidic polymers, *e.g.* polyethylene glycol (PEG), radioactive substances, toxins, low molecular weight compounds, cytokines, growth factors (e.g. TGF-$\beta$, NGF, and neurotrophin), albumins, enzymes, and other antibodies.

The terms "F(ab')$_2$" and "Fab" mean antibody fragments produced by treating an immunoglobulin with pepsin or papain which are proteases, namely produced by digestion at the upstream or downstream side of the disulfide bond existing between two heavy chains in the hinge region. For example, when IgG is treated with papain, it is cleaved at the upstream side of the disulfide bond existing between the two heavy chains in the hinge region to produce two homologous antibody fragments each comprising a light chain comprising a VL (light chain variable region) and a CL (light chain constant region) and a heavy chain fragment comprising a VH (heavy chain variable region) and a CH$\gamma$1 ($\gamma$1 region in the heavy chain constant region), in which the light chain and the heavy chain fragment are linked to each other via a disulfide bond at the C-terminal domain. Each of the two homologous antibody fragments is referred to as Fab. Meanwhile, when IgG is treated with pepsin, it is cleaved at the downstream side of the disulfide bond existing between the two heavy chains in the hinge region to produce an antibody fragment that is slightly larger than the two Fab linked to each other at the hinge region. This antibody fragment is referred to as F(ab')$_2$.

<Chimeric Antibody>

In a preferred mode of the anti-RGMa neutralizing antibody of the present invention, there is a chimeric antibody. Examples of the "chimeric antibody" include those in which the variable regions are derived from immunoglobulins from non-human animals (such as mouse, rat, hamster and chicken) and the constant regions are derived from human immunoglobulins. The chimeric antibody can be prepared, for example, by immunizing a mouse with the antigen, cleaving out a variable region that binds to the antigen from the gene encoding the mouse monoclonal antibody, and combining the variable region with a constant region of an antibody derived from human bone marrow. The constant region derived from a human immunoglobulin has a unique amino acid sequence depending on each isotype, such as IgG (IgGl, IgG2, IgG3, or IgG4), IgM, IgA (IgAl, or IgA2), IgD, or IgE. The constant region of the recombinant chimeric antibody according to the present invention may be a constant region of a human immunoglobulin belonging to any of the isotypes. The constant region is preferably a constant region of human IgG. The chimeric antibody gene thus prepared can be used to prepare an expression vector. A host cell is transformed with the expression vector to obtain a transformed cell that produces the chimeric antibody. Subsequently, the transformed cell is cultured to obtain the desired chimeric antibody from the culture supernatant.

<Humanized Antibody>

In another preferred mode of the anti-RGMa neutralizing antibody of the present invention, there is a humanized antibody. The "humanized antibody" according to the present invention is an antibody obtained by grafting only the DNA sequence of the antigen binding sites (CDRs; complementarity determining regions) of an antibody derived from a nonhuman animal such as mouse to a human antibody gene (CDR grafting). The humanized antibody can be prepared according to the methods described in, for example, Japanese Translated PCT Patent Application Laid-open No. 1992-50645 8 and Japanese Patent No. 2912618. Specifically, the humanized antibody comprises CDRs partly or wholly derived from monoclonal antibodies from non-human mammals (such as mouse, rat, and hamster); framework regions of variable regions derived from human immunoglobulins; and constant regions derived from human immunoglobulins.

The humanized antibody according to the present invention can be produced, for example, as described below. Needless to say, however, the production method is not limited thereto.

For example, a recombinant humanized antibody derived from a mouse monoclonal antibody can be produced by genetic engineering with reference to Japanese Translated PCT Patent Application Laid-open No. 1992-506458 and Japanese Laid-open Patent Application (Kokai) No. 1987-296890. Specifically, DNA encoding the region of CDRs of a mouse heavy chain and DNA encoding the region of CDRs of a mouse light chain are isolated from hybridomas producing a mouse monoclonal antibody. Further, a human heavy chain gene encoding the whole region other than CDRs of the human heavy chain and a human light chain gene encoding the whole region other than CDRs of the human light chain are isolated from a human immunoglobulin gene.

The isolated DNA encoding the region of CDRs of the mouse heavy chain is grafted to the human heavy chain gene, and the product is introduced into an appropriate expression vector so that it is expressible. Similarly, the DNA encoding the region of CDRs of the mouse light chain is grafted to the human light chain gene, and the product is introduced into another appropriate expression vector so that it is expressible. Alternatively, the human heavy and light chain genes to which mouse CDRs are grafted may be introduced into the same expression vector so that they are expressible. A host cell is transformed with the expression vector thus prepared to obtain a transformed cell producing the humanized antibody. Subsequently, the transformed cell is cultured to obtain the desired humanized antibody from the culture supernatant.

<Human Antibody>

In another preferred mode of the anti-RGMa neutralizing antibody of the present invention, there is a human antibody. The term "human antibody" refers to an antibody in which the entire region including heavy chain variable regions and heavy chain constant regions and light chain variable regions and light chain constant regions constituting the immunoglobulin are derived from genes encoding human immunoglobulins. Human antibodies can be prepared by introducing human antibody genes into mice. Human antibodies can be produced in the same manner as the above-described method for preparing polyclonal antibodies or monoclonal antibodies. Specifically, for example, the method comprises introducing at least human immunoglobulin genes into gene loci of a non-human mammal such as mouse to prepare a transgenic animal and immunizing the transgenic animal with an antigen.

For example, a transgenic mouse that produces a human antibody can be prepared according to the methods described, for example, in Nature Genetics, Vol. 7, p.13-21, 1994; Nature Genetics, Vol. 15, p.146-156, 1997; Japanese Translated PCT Patent Application Laid-open Nos. 1992-504365 and 1995-509137; WO 94/25585; Nature, Vol. 368, p.856-859, 1994; and Japanese Translated PCT Patent Application Laid-open No. 1994-500233. More specifically, for example, HuMab® Mouse (Medarex, Princeton, NJ), KMTM mouse (Kirin Pharma Company, Japan), and KM (FCγRIIb-KO) mouse may be used.

Specific examples of the anti-RGMa neutralizing antibody of the present invention include those having CDRs comprising specific amino acid sequences in the heavy chain variable region, and CDRs comprising specific amino acid sequences in the light chain variable region (preferably the anti-RGMa neutralizing antibodies (a) to (1) described above).

The amino acid sequence of the anti-RGMa neutralizing antibody (preferably the anti-RGMa neutralizing antibodies (a) to (1) described above) may have substitution, deletion, addition or insertion of one or several (1 to 20, 1 to 10, or 1 to 5, but preferably 1 or 2) amino acids as long as the antibody of the present invention maintains the characteristics of having an ability to bind to RGMa and inhibiting (neutralizing) the activity of RGMa. The substitution, deletion, or addition may be introduced in CDRs, but it is preferably introduced in a region other than CDR. The amino acid substitution is preferably conservative substitution in order to maintain the characteristics of the present invention.

When the amino acid sequence of the anti-RGMa neutralizing antibody of the present invention (preferably the anti-RGMa neutralizing antibodies (a) to (1) described above) has a substitution, deletion, or the like therein, the amino acid sequence of the heavy chain variable region after the modification of the amino acid sequence has a % identity of 90% or more (more preferably 95%, 96%, 97%, 98%, or 99% or more) to the amino acid sequence before the modification, and the amino acid sequence of the light chain variable region after the modification of the amino acid sequence has a % identity of 90% or more (more preferably 95%, 96%, 97%, 98%, or 99% or more) to the amino acid sequence before the modification.

In the present invention the term "siRNA" refers to a short double strand RNA capable of inhibiting the expression of a target gene (a RGMa gene in the present invention). The nucleotide sequence and the length (base length) are not particularly limited as long as the function as an siRNA to inhibit the RGMa activity in the present invention is maintained.

The base length is preferably less than about 30 bases, more preferably about 19 bases to 27 bases, and still more preferably about 21 bases to 25 bases.

In the present invention the term "shRNA" refers to a molecule with about 20 or more base pairs, which is a single strand RNA comprising a part having a palindromic nucleotide sequence to form a double-stranded structure in the molecule, and has a short hairpin structure with a 3'-terminal overhang. Such an shRNA can be introduced into a cell and then degraded into a length of about 20 bases (typically, for example, 21, 22, or 23 bases) in the cell to inhibit the expression of a target gene similarly to siRNA.

The siRNA and shRNA in the present invention may be in any form as long as they can inhibit the expression of the RGMa gene.

In the present invention, an siRNA or shRNA can be chemically synthesized by an artificial means. Antisense and sense RNAs can also be synthesized *in vitro* from DNA templates using, for example, a T7 RNA polymerase and a T7 promoter. The antisense oligonucleotide may be any nucleotide that is complementary to, or hybridizes to a consecutive nucleotide sequence having a length from 5 to 100 in the DNA sequence of a RGMa gene, and may be DNA or RNA. The antisense oligonucleotide may be modified insofar as its function is not impaired. The antisense oligonucleotide can be synthesized by a conventional method, and for example, it can be easily synthesized with a commercially available DNA synthesizer.

A preferred sequence can be selected by a common selection method, and the validation as the siRNA or shRNA according to the present invention can be made by evaluating inhibition of the expression of a functional RGMa.

<Acute Phase Neuromyelitis Optica>

The stages of neuromyelitis optica are clinically divided into two broad categories of "acute phase" (which is a concept including the acute exacerbation phase in the present invention) and "chronic stage".

In this regard, the "acute phase" refers to a stage where symptoms of neuromyelitis optica such as neuritis optica and myelitis appear, and these symptoms continue, or further aggravate (exacerbation). In this stage, the acute phase can be determined by reference to the finding that a gadolinium contrast effect appears in part of the lesion in an MRI inspection, and/or the finding of an increase in cell number or an elevation of protein level in an examination of cerebrospinal fluid.

On the other hand, the "chronic phase" refers to a stage where exacerbation of symptoms is subsided and the symptoms are improved or stabilized by a treatment. In such a stage the gadolinium contrast effect disappears in an MRI inspection, by which the chronic phase can be determined.

In the present invention, the acute phase of neuromyelitis optica refers to the aforementioned "acute phase", which includes not only the pathological conditions of the acute phase in the initial episode of neuromyelitis optica, but also the pathological conditions of the acute phase at the recurrence of neuromyelitis optica in the second or subsequent episode of neuromyelitis optica.

Meanwhile, it takes about 8.5 days (range: 2 days to 63 days) for neuromyelitis optica to reach its symptomatic peak in a therapeutic objective (preferably mammals, especially humans) (reference: Flanagan, et al., Ann Neurol., 2016 Mar; 79(3): 437-47, etc.). Therefore, the duration of the acute phase is usually one month or less from the onset of neuromyelitis optica.

In the present invention, neuromyelitis optica means neuromyelitis optica spectrum disorder (NMOSD) and is a concept that includes both an anti-AQP4 antibody-positive neuromyelitis optica spectrum disorder (NMOSD) and an anti-AQP4 antibodynegative neuromyelitis optica spectrum disorder (NMOSD), which are listed in the international consensus diagnostic criteria for neuromyelitis optica (Wingerchuk, et al., Neurology, 2015; 8582: 177-189). Among others, the present invention is suitable for an anti-AQP4 antibody-positive neuromyelitis optica spectrum disorder.

The therapeutic objective (preferably a mammal, especially a human) of the present invention is a patient who has developed a neuromyelitis optica spectrum disorder (NMOSD), preferably a patient who has developed an anti-AQP4 antibody-positive neuromyelitis optica spectrum disorder (NMOSD), and an agent for preventing or treating acute phase neuromyelitis optica of the present invention can be administered to such a patient.

Since acute phase neuromyelitis optica in the present invention is often accompanied by pains as one of the cardinal symptoms of the patient, according to the present invention the RGMa inhibiting substance, preferably the anti-RGMa neutralizing antibody may be used as an agent for preventing or treating the pain symptoms seen in neuromyelitis optica on a patient suffering from such pains.

The matters described in connection with the preventive or therapeutic agent or the preventive or therapeutic method for acute phase neuromyelitis optica of the present invention are entirely applicable to the description of a preventive or therapeutic agent or a preventive or therapeutic method for pain symptoms seen in neuromyelitis optica of the present invention.

In this regard, a "treatment" includes any treatment of a disease in a therapeutic objective, preferably a mammal, and especially a human, and includes prevention of progression of a disease or symptom so as to extinguish, heal, alleviate, or mitigate such a disease and symptom.

The term "prevention" also includes prevention or inhibition of the onset of the above diseases in a therapeutic objective, preferably a mammal, and especially a human. Furthermore, "prevention" in the present invention includes "recurrence prevention" which prevents recurrence of the above-mentioned diseases in which remission and recurrence are repeated in a therapeutic objective, preferably a mammal, and especially a human.

<Pharmaceutical composition>

The agent for preventing or treating acute phase neuromyelitis optica in the present invention is usually administered systemically or locally in an oral or parenteral form.

The agent for preventing or treating acute phase neuromyelitis optica in the present invention can be made into a formulation by blending a pharmaceutically acceptable carrier or additive as appropriate using a RGMa inhibiting substance as an active ingredient. The thus formulated pharmaceutical composition can be administered in an oral or parenteral form. Specifically, the agent can be formed into oral agents such as tablets, coated tablets, pills, powders, granules, capsules, solutions, suspensions, and emulsions; or parenteral agents such as injections, infusions, suppositories, ointments, and patches. The content ratio of the carrier or additive may be set as appropriate according to the ranges commonly used in the pharmaceutical field. There is no particular restriction on the carrier or additive to be blended, and examples thereof include water, physiological saline, other aqueous solvents, various carriers such as aqueous or oily bases; and various additives such as excipients, binders, pH adjusters, disintegrants, absorption promoters, lubricants, colorants, flavoring agents, and perfumes.

When the RGMa inhibiting substance is an anti-RGMa neutralizing antibody, a functionally modified antibody thereof, a conjugated antibody thereof, or an antigen-binding fragment thereof, the agent is preferably formulated into an injection or infusion with a pharmaceutically acceptable carrier, and administered via a parenteral route of administration, such as an intravenous, intramuscular, intradermal, intraperitoneal, subcutaneous, or local route.

The injection or infusion containing the anti-RGMa neutralizing antibody can be, for example, used as a solution, suspension, or emulsion. Examples of the solvent therefor include distilled water for injection, physiological saline, a glucose solution, and an isotonic solution (for example, solutions of sodium chloride, potassium chloride, glycerin, mannitol, sorbitol, boracic acid, borax, and propylene glycol).

The injections or infusions containing the anti-RGMa neutralizing antibody may further contain a stabilizer, a solubilizer, a suspending agent, an emulsifier, a soothing agent, a buffer agent, a preservative, an antiseptic, a pH adjuster, etc.

Examples of the stabilizer include albumin, globulin, gelatin, mannitol, glucose, dextran, ethylene glycol, propylene glycol, ascorbic acid, sodium bisulfite, sodium thiosulfate, sodium EDTA, sodium citrate, and dibutylhydroxytoluene.

Examples of the solubilizer include alcohols (such as ethanol), polyalcohols (such as propylene glycol and polyethylene glycol), and nonionic surfactants (such as polysorbate 80 (registered trademark), and HCO-50).

Examples of the suspending agent include glyceryl monostearate, aluminum monostearate, methylcellulose, carboxymethylcellulose, hydroxymethylcellulose, and sodium lauryl sulfate.

Examples of the emulsifier include gum arabic, sodium alginate, and tragacanth.

Examples of the soothing agent include benzylalcohol, chlorobutanol, and sorbitol.

Examples of the buffer agent include a phosphate buffer, acetate buffer, borate buffer, carbonate buffer, citrate buffer, and Tris buffer.

Examples of the preservative include methyl *para*-hydroxybenzoate, ethyl *para*-hydroxybenzoate, propyl *para*-hydroxybenzoate, butyl *para*-hydroxybenzoate, chlorobutanol, benzyl alcohol, benzalkonium chloride, sodium dehydroacetate, sodium edetate, boric acid, and borax.

Examples of the antiseptic include benzalkonium chloride, *para*-hydroxybenzoic acid, and chlorobutanol.

Examples of the pH adjuster include hydrochloric acid, sodium hydroxide, phosphoric acid, and acetic acid.

When the RGMa inhibiting substance is a nucleic acid (such as siRNA, shRNA, and antisense oligonucleotide), it can be administered in the form of a nonviral or viral vector. When it is in the form of a nonviral vector, a method of introducing a nucleic acid molecule using a liposome (such as a liposome method, an HVJ-liposome method, a cationic liposome method, a lipofection method, or a lipofectamine method), a microinjection method, a method of transferring a nucleic acid molecule with a carrier (a metal particle) into a cell using a gene gun or the like, can be used. When siRNA or shRNA is administered to a living organism using a viral vector, a viral vector such as a recombinant adenovirus or retrovirus can be used. A DNA that expresses the siRNA or shRNA is introduced to a DNA virus or RNA virus such as detoxified retrovirus, adenovirus, adeno-associated virus, herpesvirus, vaccinia virus, poxvirus, poliovirus, sindbis virus, Sendai virus, or SV40. By infecting a cell or a tissue with the recombinant virus, the gene can be introduced into the cell or tissue.

The formulation thus obtained can be administered to, for example, a human or another mammal (such as a rat, mouse, rabbit, sheep, pig, cattle, cat, dog, or monkey) at an effective dose to prevent or treat acute phase neuromyelitis optica. The dose is set as appropriate depending on the purpose, the severity of a disease, the age, weight, sex, and past history of a patient, the type of an active ingredient, or the like. For example, when the active ingredient is an anti-RGMa neutralizing antibody, the dose for an average human having a weight of about 65 kg to 70 kg is preferably about 0.02 mg to 4000 mg per day, more preferably about 0.1 mg to 200 mg per day. The total dose per day may be administered in a single dose or in divided doses.

<Combination with Other Drug or Treatment>

In the present invention, an agent for preventing or treating acute phase neuromyelitis optica may be administered in combination with another drug or treatment useful for treating neuromyelitis optica.

Examples of other drug or treatment that may be used in combination with an agent for preventing or treating acute phase neuromyelitis optica according to the present invention include plasma exchange and/or intravenous administration of an immunoglobulin preparation, and administration of mycophenolate, rituximab, eculizumab, eculizumab, and/or satralizumab. Such other drugs or treatment may be any other biologically active drug or treatment that is effective in treating a central nervous system disorder such as neuromyelitis optica, or in delaying progression of a central nervous system disorder.

For example, such other biologically active drugs may be corticosteroid, an (intravenous) immunoglobulin preparation, or an anti-lymphocyte preparation, mycophenolate, rituximab, eculizumab and/or satralizumab. In a preferred embodiment, a patient is treated by intravenous immunotherapy (e.g., a corticosteroid, for example, a (synthetic) glucocorticoid, such as methylprednisolone). Therefore, such other biologically active drug may be a corticosteroid, for example, a (synthetic) glucocorticoid, such as methylprednisolone. Such other biologically active drug is intravenously administered. Tn a case where a patient is refractory to steroids (for example, in a case where only inadequate suppression of inflammation in the central nervous system is recognized after receiving a steroid treatment), in connection with such other drug or treatment, plasma exchange may be performed. Therefore, a patient, including a patient refractory to steroids, may optionally undergo plasma exchange.

The above other drug or treatment may be administered or performed before or after administration of an agent for preventing or treating acute phase neuromyelitis optica according to the present invention, or may be administered or performed at the same time.

EXAMPLES

The present invention will be described in more detail below with reference to Examples, which do not limit the scope of the present invention.

[Example 1] Effect of Anti-RGMa Neutralizing Antibody on Acute Phase Neuromyelitis Optica

The therapeutic effect of an anti-RGMa neutralizing antibody on exacerbation of clinical symptom was evaluated using an acute phase of severe experimental NMO rat model. As an anti-RGMa neutralizing antibody, an anti-RGMa neutralizing antibody including the amino acid sequence of (a) (SEQ TD NOS: 5 to 10) described herein was used in the experiment.

(1-1) Procedure of NMO induction in rats

An acute phase of severe experimental NMO rat model was produced according to a previous report (Kurosawa K, et al., Acta Neuropathol Commun. 2015; 3:82). Female Lewis rats were used in the experiment. The immunization to central nervous antigen MBP was introduced as a stimulus for proinflammatory environmental induction and disruption of the blood-spinal cord barrier (BSCB). A myelin basic protein from guinea pig brain was used as MBP, the MBP was dissolved in PBS to 1 mg/mL, mixed in equal amounts with a complete Freund's adjuvant containing 1 mg/mL of killed Mycobacterium tuberculosis H37Ra, and sonicated to prepare an emulsion. The prepared emulsion (200 μL/head) was administered subcutaneously at two points in the dorsal region. On day 10 after MBP injection, animals with a neurological symptom score of 1 or less were administered a single intraperitoneal administration of an anti-AQP4 antibody (murine anti-AQP4 monoclonal antibody E5415A) at a dose of 3 mg/kg to induce an anti-AQP4 antibody-dependent exacerbation of clinical symptom.

(1-2) Evaluation of Neurological Symptom Score

The neurological symptoms were scored based on the following criteria, and the sum of the scores for the tail and each hindlimb was used for the analysis as neurological symptom score (scored from 0 to 6). Scores regarding the tail; 0: no signs of paresis 1: paresis, and 2: Complete paralysis; scores regarding each hind limb; 0: no signs of paresis 1: paresis, and 2: the hindlimb is paralyzed and dragged. The neurological symptoms were scored in a blinded manner once every day until day 13 after the administration of the anti-RGMa neutralizing antibody (14th day of the administration of the anti-AQP4 antibody).

(1-3) Grouping and Administration of Anti-RGMa Neutralizing Antibody

The rats were divided into two groups so that bias in the mean values of neurological symptom score and body weight on day 1 after the administration of anti-AQP4 antibody were minimized between the two groups. An anti-RGMa neutralizing antibody or an isotype control antibody (palivizumab) was administered intravenously at a single dose of 10 mg/kg into the tail vein. Each of the anti-RGMa neutralizing antibody administration group and the isotype control antibody administration group was consisting of six animals.

(1-4) Results

The effect of a single administration of an anti-RGMa neutralizing antibody on exacerbation of clinical symptom in an acute phase of severe experimental NMO rat model is shown in Fig. 1. On the next day of the administration of the anti-AQP4 antibody, an anti-RGMa neutralizing antibody or an isotype control antibody was administered intravenously, and neurological symptoms were evaluated once daily until day 14 after the administration of the anti-AQP4 antibody. The neurological symptom score of the group that received the anti-RGMa neutralizing antibody on the next day of the administration of the anti-AQP4 antibody was lower than the group that received the isotype control antibody throughout the observation period. The average scores during the periods from day 2 to day 8, and from day 2 to day 14 after the administration of the anti-AQP4 antibody were lowered significantly (the average score during the period from day 2 to day 8 of $2.13\pm0.41$ vs. $3.70\pm0.20$ for isotype control IgG-treated rats, $p < 0.01$ in Mann-Whitney U test; and the average score during the period from day 2 to day 14 of $1.90\pm0.43$ vs. $3.05\pm0.19$ for isotype control IgG-treated rats, $p < 0.05$ in Mann-Whitney U test).

From the above results, it has become clear that the RGMa inhibiting substance, especially the anti-RGMa neutralizing antibody, exhibits therapeutic effect on clinical exacerbation in acute phase neuromyelitis optica.

[Example 2] Effect of Anti-RGMa Neutralizing Antibody on Blood Spinal Cord Barrier Disruption

The therapeutic effect of an anti-RGMa neutralizing antibody on BSCB disruption was examined by gadolinium contrast enhanced MRI using tEAE mice which locally induced BSCB disruption in the spinal cord. As the anti-RGMa neutralizing antibody, an anti-RGMa neutralizing antibody including the amino acid sequence of (a) (SEQ ID NOS: 5 to 10) described herein was used in the experiment.

(2-1) Production of tEAE mice

Female C57/BL6J mice were used. Myelin Oligodendrocyte Glycoprotein Peptide Fragment 35-55, rat, mouse (MEVGWYRSPFSRVVHLYRNGK (SEQ ID NO: 46); $MOG_{35-55}$, Sigma- Aldrich) was dissolved in PBS to 2 mg/mL, mixed in equal amounts with a complete Freund's adjuvant containing 5 mg/mL of killed Mycobacterium tuberculosis H37Ra, and sonicated to prepare an emulsion. Each 100 $\mu$L of the prepared emulsion (200 $\mu$L/head) was administered subcutaneously at two points in the dorsal region to introduce MOG immunity. Approximately 21 days later, 1.5 $\mu$L of a cytokine mixture solution (750 ng of Tumor Necrosis Factor-a, and 1 $\mu$g of Interferon-y) was injected into the thoracic spinal cord to the depth of 0.5 mm to 0.8 mm below the eighth thoracic vertebra, and immediately thereafter and 2 days later, 200 ng of pertussis toxin was administered into the tail vein to induce tEAE.

(2-2) Evaluation of Neurological symptom Score

The neurological symptom score was evaluated according to the aforedescribed criteria (see Tanabe S, Fujita Y, Tkuma K, Yamashita T., Inhibiting repulsive guidance molecule A suppresses secondary progression in mouse models of multiple sclerosis, Cell Death Dis. 2018; 9(11):1061) in a blinded manner.

(2-3) Evaluation of BSCB Disruption Using Gadolinium Contrasted Magnetic Resonance Imaging (MRI)

Sequential T1-weighted images (dynamic contrast-enhanced MRT; DCE-MRT) before and after the administration of a gadolinium contrast agent (Omniscan, Daiichi Sankyo) were taken using a BioSpec 117/11 (Bruker Corporation), and the leakage of gadolinium into the spinal cord, which was an indicator of BSCB disruption, was quantitatively assessed from the intensity change of the T1 signal. Under sevoflurane anesthesia and with a body temperature maintenance device, a gadolinium contrast agent was administered rapidly at a dose of 0.25 mmol/kg via an indwelling catheter tube in the tail vein. In performing DCE-MRI, a 200×200 acquisition matrix was set with respect to a 26×26 mm effective field of view (FoV) centered on the cytokine infusion point of the thoracic spinal cord, and images of 11 axial planes with 0.8 mm slice thickness and interval were acquired. In this regard, the repetition time (TR) was set at 500 ms, the echo time (TE) was set at 18 ms, and the number of excitation (NEX) was set at 4, and total 6 images were taken sequentially over about 10 minutes (about 100 sec per image).

The images were output in the DICOM format, and analyzed using Fiji (http://fiji.sc/). In order to exclude the effect of T1 signals of the cerebrospinal fluid, a region of interest (ROI) was defined in the spinal cord, and the T1 signal enhancement ratio (SER) in the spinal cord at each time point was calculated based on Equation (1). The intensity of gadolinium intraspinal leakage (total Gd influx rate) for each individual was calculated as set forth in Equation (2) by determining the gradient with respect to time as gadolinium influx rate using the SLOPE function in Microsoft Excel 2016 (Microsoft), and totalizing the values of 11 slices.

$$\text{SER (t)} = \frac{\text{T1 signal post Gd injection (t)} - \text{T1 signal pre Gd injection}}{\text{T1 signal pre Gd injection}} \quad \cdots (1)$$

$$\text{Total Gd influx rate} = \sum_{Slice\,1}^{Slice\,11} SLOPE(SER(0):SER(10),0:10) \quad \cdots (2)$$

Based on the results of the study on normal mice, when the value of SLOPE (SER (0): SER (10), 0:10) in Equation (2) was less than 0.02, this value was deemed as an analytical error and excluded from summation according to Equation (2).

(2-4) Grouping and Administration of Anti-RGMa Neutralizing Antibody

The mice were divided into two so that the measured quantities of gadolinium leakage on day 7 after the cytokine injection became even between the two groups. An anti-RGMa neutralizing antibody or an isotype control antibody (palivizumab) was administered twice a week at a dose of 10 mg/kg into the tail vein. On each individual in the anti-RGMa neutralizing antibody-treated group consisting of 10 animals and the isotype control antibody-treated group consisting of 9 animals, the assessment of the neurological symptom score and the MRI analysis were performed.

(2-5) Results

The recovering effect of an anti-RGMa neutralizing antibody on the BSCB disruption and neurological symptoms in the acute phase of tEAE mice is shown in Fig. 2. The recovering effect of an anti-RGMa neutralizing antibody on the BSCB disruption was analyzed by calculating the alteration of gadolinium leakage level on 7, 14, and 21 days after the cytokine injection relative to the gadolinium leakage level on 7 days after cytokine injection. The leakage of gadolinium into the spinal cord that was occurring on day 7 after the cytokine infusion was remarkably suppressed by repeated administrations of the anti-RGMa neutralizing antibody (on day 14 after cytokine infusion, p < 0.001, Bonferroni multicomparison test) and early recovery for BSCB disruption was observed (Fig. 2, Panel A). Further, the neurological symptom score obtained in the same individual was also significantly suppressed, and early recovery of neurological symptoms was also recognized (Fig. 2, Panel B).

The correlation between the level of gadolinium leakage into spinal cord and the severity of neurological symptoms in the acute phase of tEAE mice is shown in Fig. 3. There was a strong positive correlation (r = 0.831, p < 0.001) between the level of gadolinium leakage into spinal cord and the severity of neurological symptoms on 7 days after the cytokine injection, and there was also a recognizable positive correlation on day 14 (r = 0.549, p < 0.05). It was shown that the severity of BSCB disruption defined the severity of neurological symptoms.

These results indicate that a RGMa inhibiting substance, especially an anti-RGMa neutralizing antibody, exhibits a pharmaceutical effect on acute phase neuromyelitis optica by a recovery acceleratory effect on BSCB disruption.

[Example 3] Therapeutic Effect of Anti-RGMa Neutralizing Antibody on the Blood Spinal Cord Barrier Disruption in Acute Phase Neuromyelitis Optica

Intraspinal leakage of rat TgG due to blood-spinal barrier disruption was analyzed by immunohistochemical staining to evaluate the therapeutic effect of anti-RGMa neutralizing antibody on blood-spinal barrier disruption in acute phase of severe experimental NMO rat model.

(3-1) Preparation of acute phase of severe NMO rat model

Female Lewis rats were used for the experiment. On day 10 after MBP immunization, individuals with a neurological symptom score of 1 or less were selected and a single intraperitoneal administration of the anti-AQP4 antibody (mouse anti-AQP4 monoclonal antibody E5415A) was performed at a dose of 3 mg/kg to induce NMO pathology.

(3-2) Grouping and administration of the neutralizing antibody

The animals were divided into two groups so that bias in the mean values of neurological symptom score and body weight on day 1 after the administration of anti-AQP4 antibody were minimized between the two groups, and the anti-RGMa neutralizing antibody or isotype control antibody (Palivizumab) was administered intravenously at a dose of 10 mg/kg. The anti-RGMa neutralizing antibody-treated group and isotype control antibody-treated group consisted of 6 animals each, and the healthy untreated group consisted of 4 animals.

(3-3) Immunohistochemical staining

Dissection was performed on day 4 after administration of anti-AQP4 antibody. After exsanguination, the spinal cord was taken and immersion-fixed in 4% paraformaldehyde at 4°C for 1 day. After post-fixation and cryoprotection with sucrose replacement, the tissue pieces were embedded in OCT compound, thinly sliced at 30 $\mu$m to produce frozen sections, and immunohistochemistry was performed using Alxa488-labeled donkey anti-rat IgG antibody (1:500, Thermo Fisher Scientific). The stained sections were imaged using an Olympus TX83 inverted fluorescence microscope, and the percentage of rat IgG-positive area in the spinal cord cross-section (% rat IgG-positive area) was measured using Image J software for image analysis.

(3-4) Results

The effect of the anti-RGMa neutralizing antibody on spinal cord leakage of rat IgG is shown in Fig. 4. The administration of the anti-RGMa neutralizing antibody on the next day after the onset of NMO significantly suppressed intraspinal leakage of rat IgG, an indicator of blood-spinal cord barrier disruption.
These results suggest that the effects of RGMa inhibiting substance, especially anti-RGMa neutralizing antibody on acute phase neuromyelitis optica include the effect of promoting the restoration against blood-spinal cord barrier disruption.

[Example 4] Effect of Anti-RGMa Neutralizing Antibody on Pain Symptom due to NMO

Acute and severe experimental NMO rats were repeatedly administered with the anti-RGMa neutralizing antibody to evaluate the effect of anti-RGMa neutralizing antibody on prolonged pain.

(4-1) Preparation of an acute and severe NMO rat model

Female Lewis rats were used for the experiment. On day 10 after MBP immunization, individuals with a neurological symptom score of 1 or less were selected and a single intraperitoneal administration of the anti-AQP4 antibody (mouse anti-AQP4 monoclonal antibody E5415A) was performed at a dose of 3 mg/kg to induce NMO pathology.

(4-2) Evaluation of pain-related behaviors

For the pain evaluation, the up-down method with von Frey stimulation (Chaplan, S.R., Bach, F.W., Pogrel, J.W., Chung, J.M., Yaksh, T.L., Quantitative assessment of tactile allodynia in the rat paw, J. Neurosci. Methods, 53, 55-63 (1994)) was used to determine the 50% paw withdrawal threshold (g) for hindlimb elevation. The mean of the 50% paw withdrawal thresholds (g) of bilateral hind limbs was used for the analysis.

(4-3) Grouping and the anti-RGMa neutralizing antibody administration

The animals were divided into two groups so that bias in the mean values of neurological symptom score and body weight on day 1 after the administration of anti-AQP4 antibody were minimized between the two groups., and anti-RGMa neutralizing antibody or isotype control antibody (Palivizumab) was administered intravenously once a week at a dose of 10 mg/kg. The analysis group consisted of three groups: anti-RGMa neutralizing antibody-treated group, isotype control antibody-treated group, and healthy untreated group, all with six animals. In the exacerbation period of neurological symptoms, some animals were unable to perform von Frey stimulation due to hindlimb weakness. Therefore, the data set consisted of 5 animals in the anti-RGMa neutralizing antibody-treated group on day 4 after anti-AQP4 antibody administration, 2 animals in the isotype control antibody-treated group and 3 animals in the anti-RGMa neutralizing antibody-treated group on day 7 after anti-AQP4 antibody administration.

(4-4) Results

The inhibitory effect of the anti-RGMa-neutralizing antibody on pain-related behaviors is shown in Fig. 5. The group treated with anti-RGMa-neutralizing antibody from the next day after the NMO onset showed faster recovery from the 50% paw withdrawal threshold decrease than the group treated with isotype control antibody, and a significant increase in the 50% paw withdrawal threshold was observed on days 18 and 21 after treatment with anti-AQP4 antibody.
These results suggest that the RGMa inhibiting substance, especially anti-RGMa neutralizing antibody, has a therapeutic effect on pain symptoms in neuromyelitis optica.

[Example 5] Inhibitory Effect of Anti-RGMa Neutralizing Antibody on Granulocyte Infiltration in Spinal Cord

The inhibitory effect of the anti-RGMa neutralizing antibody on granulocyte infiltration in the spinal cord of acute phase of severe experimental NMO rat model was analyzed by immunohistochemical staining.

(5-1) Preparation of acute phase of severe NMO rat model

Female Lewis rats were used for the experiment. On day 10 after MBP immunization, individuals with a neurological symptom score of 1 or less were selected and a single intraperitoneal administration of the anti-AQP4 antibody (mouse anti-AQP4 monoclonal antibody E5415A) was performed at a dose of 3 mg/kg to induce NMO pathology.

(5-2) Grouping and the anti-RGMa antibody administration

The animals were divided into two groups so that bias in the mean values of neurological symptom score and body weight on day 1 after the administration of anti-AQP4 antibody were minimized between the two groups., and the anti-RGMa neutralizing antibody or isotype control antibody (Palivizumab) was administered intravenously at a dose of 10 mg/kg. The anti-RGMa neutralizing antibody-treated group and isotype control antibody-treated group consisted of 6 animals each, and the healthy untreated group consisted of 4 animals.

(5-3) Immunohistochemical staining

Dissection was performed on day 4 after administration of the anti-AQP4 antibody. After exsanguination, the spinal cord was taken and immersion-fixed in 4% paraformaldehyde at 4°C for 1 day. After post-fixation, the tissue fragments were cryoprotected by sucrose substitution and embedded in OCT compound thinly sliced at 30 $\mu$m to produce frozen sections. Immunohistochemistry was performed using rabbit anti-rat granulocyte serum (1:5000, LifeSpan BioSciences) as the primary antibody and Alxa488-conjugated donkey anti-rabbit antibody (1:500, Thermo Fisher Scientific) as the secondary antibody. The stained sections were imaged using an Olympus TX83 inverted fluorescence microscope, and the percentage of granulocyte-positive area (% granulocyte-positive area) was measured using Image J software for image analysis.

(5-4) Results

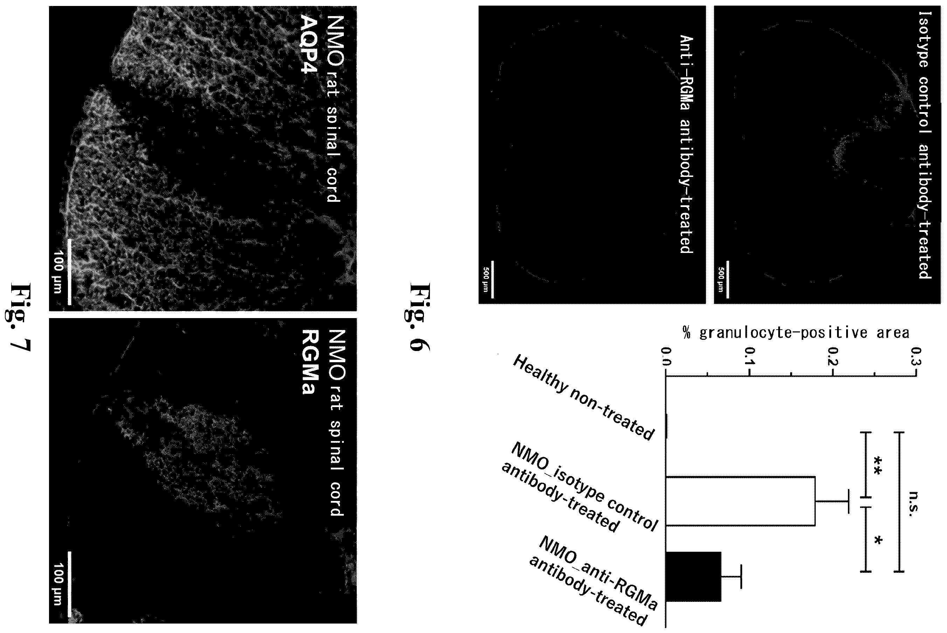

The inhibitory effect of the anti-RGMa neutralizing antibody on granulocyte infiltration in the spinal cord of the acute phase of severe experimental NMO rat model is shown in Fig. 6. The administration of the anti-RGMa neutralizing antibody on the next day after the onset of NMO significantly inhibited granulocyte infiltration in the spinal cord of NMO rats.
From these results, it was found that the RGMa inhibiting substance, especially anti-RGMa neutralizing antibody, suppresses the granulocyte infiltration observed in the pathology of acute phase neuromyelitis optica. Since the granulocyte infiltrationsuppressing effect is considered to contribute to the effect on neuromyelitis optica, it was suggested that the RGMa inhibiting substance, especially anti-RGMa neutralizing antibody, exhibits the effect on neuromyelitis optica through the granulocyte infiltrationsuppressing effect.

[Example 6] RGMa Expression at the AQP4 Loss Sites in Spinal Cord

Immunohistochemical staining of NMO rat spinal cord was performed to detect the expression of RGMa in acute phase of severe experimental NMO rat model.

(6-1) Preparation of acute phase of severe NMO rat model

Female Lewis rats were used for the experiment. Myelin basic protein from guinea pig brain was dissolved in PBS to 1 mg/mL and mixed with an equal volume of Freund's complete adjuvant containing 1 mg/mL of killed Mycobacterium tuberculosis H37Ra to produce an emulsion by sonication. MBP emulsion (200 μl/head) was administered subcutaneously (MBP immunization), and 10 days later, individuals with a neurological symptom score of 1 or less were selected and a single intraperitoneal administration of anti-AQP4 antibody (mouse anti-AQP4 monoclonal antibody E5415A) was performed at a dose of 3 mg/kg to induce NMO pathology.

(6-2) Immunohistochemical staining

Dissection was performed on day 1 after administration of anti-AQP4 antibody. After exsanguination, the spinal cord was taken and immersion-fixed in 4% paraformaldehyde at 4°C for 1 day. After post-fixation and cryoprotection by sucrose substitution, the tissue pieces were embedded in OCT compound and thinly sliced at 30 μm to produce frozen sections, which were then stained with goat anti-RGMa antibody (1:100, R&D) and rabbit anti-AQP4 antibody (1:1000, Cell Signaling Technology) as primary antibodies, and Alxa488-labeled donkey anti-goat TgG antibody (1 :500, Thermo Fisher Scientific) and Alxa647-labeled donkey anti-rabbit IgG antibody (1:500, Thermo Fisher Scientific) as secondary antibodies for double immunohistochemical staining. An Olympus IX83 inverted fluorescence microscope was used to image the stained sections.

(6-3) Results

Immunohistochemical staining images of the spinal cord sections from the acute phase of severe experimental NMO rat model are shown in Fig. 7, which show strong expression of RGMa at the AQP4 loss sites, i.e., an NMO lesion sites.

<Sequence Listing>

SEQ ID NO: 1: Amino acid sequence of human RGMa precursor protein

SEQ ID NO: 2: Amino acid sequence of murine RGMa precursor protein

SEQ ID NO: 3: Amino acid sequence of rat RGMa precursor protein

SEQ ID NO: 4: DNA sequence of human RGMa gene

SEQ ID NO: 5: Amino acid sequence of LCDR1 of anti-RGMa neutralizing antibody r116A3

SEQ ID NO: 6: Amino acid sequence of LCDR2 of anti-RGMa neutralizing antibody r116A3

SEQ ID NO: 7: Amino acid sequence of LCDR3 of anti-RGMa neutralizing antibody r116A3

SEQ ID NO: 8: Amino acid sequence of HCDR1 of anti-RGMa neutralizing antibody r116A3

SEQ ID NO: 9: Amino acid sequence of HCDR2 of anti-RGMa neutralizing antibody r116A3

SEQ ID NO: 10: Amino acid sequence of HCDR3 of anti-RGMa neutralizing antibody r116A3

SEQ ID NO: 11: Amino acid sequence of LCDR1 of anti-RGMa neutralizing antibody r70E

SEQ ID NO: 12: Amino acid sequence of LCDR2 of anti-RGMa neutralizing antibody r70E

SEQ ID NO: 13: Amino acid sequence of LCDR3 of anti-RGMa neutralizing antibody r70E

SEQ ID NO: 14: Amino acid sequence of HCDR1 of anti-RGMa neutralizing antibody r70E

SEQ ID NO: 15: Amino acid sequence of HCDR2 of anti-RGMa neutralizing antibody r70E

SEQ ID NO: 16: Amino acid sequence of human RGMa epitope

SEQ ID NO: 17: Amino acid sequence of LCDR1 of anti-RGMa neutralizing antibody 5F9

SEQ ID NO: 18: Amino acid sequence of LCDR2 of anti-RGMa neutralizing antibody 5F9

SEQ ID NO: 19: Amino acid sequence of LCDR3 of anti-RGMa neutralizing antibody 5F9

SEQ ID NO: 20: Amino acid sequence of HCDR1 of anti-RGMa neutralizing antibody 5F9

SEQ ID NO: 21: Amino acid sequence of HCDR2 of anti-RGMa neutralizing antibody 5F9

SEQ ID NO: 22: Amino acid sequence of HCDR3 of anti-RGMa neutralizing antibody 5F9

SEQ ID NO: 23: Amino acid sequence of LCDR1 of anti-RGMa neutralizing antibody 8D1

SEQ ID NO: 24: Amino acid sequence of LCDR2 of anti-RGMa neutralizing antibody 8D1

SEQ ID NO: 25: Amino acid sequence of LCDR3 of anti-RGMa neutralizing antibody 8D1

SEQ ID NO: 26: Amino acid sequence of HCDR1 of anti-RGMa neutralizing antibody 8D1

SEQ ID NO: 27: Amino acid sequence of HCDR2 of anti-RGMa neutralizing antibody 8D1

SEQ ID NO: 28: Amino acid sequence of HCDR3 of anti-RGMa neutralizing antibody 8D1

SEQ ID NO: 29: Amino acid sequence of LCDR1 of anti-RGMa neutralizing antibody AE12-1

SEQ ID NO: 30: Amino acid sequence of LCDR2 of anti-RGMa neutralizing antibody AE12-1

SEQ ID NO: 31: Amino acid sequence of LCDR3 of anti-RGMa neutralizing antibody AE12-1

SEQ ID NO: 32: Amino acid sequence of HCDR1 of anti-RGMa neutralizing antibody AE12-1

SEQ ID NO: 33: Amino acid sequence of HCDR2 of anti-RGMa neutralizing antibody AE12-1

SEQ ID NO: 34: Amino acid sequence of HCDR3 of anti-RGMa neutralizing antibody AE12-1

SEQ ID NO: 35: Amino acid sequence of LCDR3 of anti-RGMa neutralizing antibody AE12-1Y

SEQ ID NO: 36: Amino acid sequence of human RGMa epitope

SEQ ID NO: 37: Amino acid sequence of human RGMa epitope

SEQ ID NO: 38: Amino acid sequence of human RGMa epitope

SEQ ID NO: 39: Amino acid sequence of human RGMa epitope

SEQ ID NO: 40: Amino acid sequence of LCDR3 of anti-RGMa neutralizing antibody AE12-1F

SEQ ID NO: 41: Amino acid sequence of LCDR3 of anti-RGMa neutralizing antibody AE12-1H

SEQ ID NO: 42: Amino acid sequence of LCDR3 of anti-RGMa neutralizing antibody AE12-1L

SEQ TD NO: 43: Amino acid sequence of LCDR3 of anti-RGMa neutralizing antibody AE12-1V

SEQ ID NO: 44: Amino acid sequence of LCDR3 of anti-RGMa neutralizing antibody AE12-1I

SEQ ID NO: 45: Amino acid sequence of LCDR3 of anti-RGMa neutralizing antibody AE12-1K

SEQ ID NO: 46: Amino acid sequence (35-55) of rat, mouse MOG

Industrial Applicability

Since the RGMa inhibiting substance is useful for preventing or treating acute phase neuromyelitis optica, and also useful for preventing or treating pain symptoms in

**Claims**

1.  An agent for preventing or treating acute phase neuromyelitis optica comprising a RGMa inhibiting substance.

2.  The preventive or therapeutic agent according to claim 1, wherein the RGMa inhibiting substance is an anti-RGMa neutralizing antibody.

3.  The preventive or therapeutic agent according to claim 2, wherein the anti-RGMa neutralizing antibody is a humanized antibody.

4.  The preventive or therapeutic agent according to claim 2 or 3, wherein the anti-RGMa neutralizing antibody is an antibody recognizing an amino acid sequence selected from SEQ ID NOS: 16, 36, 37, 38 and 39.

5.  The preventive or therapeutic agent according to any one of claims 2 to 4, wherein the anti-RGMa neutralizing antibody is an antibody selected from the following (a) to (1):

    (a) an anti-RGMa neutralizing antibody comprising a light chain variable region comprising an LCDR1 comprising the amino acid sequence represented by SEQ ID NO: 5, an LCDR2 comprising the amino acid sequence represented by SEQ ID NO: 6, and an LCDR3 comprising the amino acid sequence represented by SEQ ID NO: 7, and a heavy chain variable region comprising an HCDR1 comprising the amino acid sequence represented by SEQ TD NO: 8, an HCDR2 comprising the amino acid sequence represented by SEQ ID NO: 9, and an HCDR3 comprising the amino acid sequence represented by SEQ ID NO: 10;
    (b) an anti-RGMa neutralizing antibody comprising a light chain variable region comprising an LCDR1 comprising the amino acid sequence represented by SEQ ID NO: 11, an LCDR2 comprising the amino acid sequence represented by SEQ ID NO: 12, and an LCDR3 comprising the amino acid sequence represented by SEQ ID NO: 13, and a heavy chain variable region comprising an HCDR1 comprising the amino acid sequence represented by SEQ TD NO: 14, an HCDR2 comprising the amino acid sequence represented by SEQ ID NO: 15, and an HCDR3 comprising an amino acid sequence comprising SFG;
    (c) an anti-RGMa neutralizing antibody comprising a light chain variable region comprising an LCDR1 comprising the amino acid sequence represented by SEQ ID NO: 17, an LCDR2 comprising the amino acid sequence represented by SEQ ID NO: 18, and an LCDR3 comprising the amino acid sequence represented by SEQ TD NO: 19, and a heavy chain variable region comprising an HCDR1 comprising the amino acid sequence represented by SEQ ID NO: 20, an HCDR2 comprising the amino acid sequence represented by SEQ ID NO: 21, and an HCDR3 comprising the amino acid sequence represented by SEQ ID NO: 22;
    (d) an anti-RGMa neutralizing antibody comprising a light chain variable region comprising an LCDR1 comprising the amino acid sequence represented by SEQ ID NO: 23, an LCDR2 comprising the amino acid sequence represented by SEQ ID NO: 24, and an LCDR3 comprising the amino acid sequence represented by SEQ ID NO: 25, and a heavy chain variable region comprising an HCDR1 comprising the amino acid sequence represented by SEQ ID NO: 26, an HCDR2 comprising the amino acid sequence represented by SEQ ID NO: 27, and an HCDR3 comprising the amino acid sequence represented by SEQ ID NO: 28;

(e) an anti-RGMa neutralizing antibody comprising a light chain variable region comprising an LCDR1 comprising the amino acid sequence represented by SEQ ID NO: 29, an LCDR2 comprising the amino acid sequence represented by SEQ ID NO: 30, and an LCDR3 comprising the amino acid sequence represented by SEQ ID NO: 31, and a heavy chain variable region comprising an HCDR1 comprising the amino acid sequence represented by SEQ ID NO: 32, an HCDR2 comprising the amino acid sequence represented by SEQ ID NO: 33, and an HCDR3 comprising the amino acid sequence represented by SEQ ID NO: 34;

(f) an anti-RGMa neutralizing antibody comprising a light chain variable region comprising an LCDR1 comprising the amino acid sequence represented by SEQ ID NO: 29, an LCDR2 comprising the amino acid sequence represented by SEQ ID NO: 30, and an LCDR3 comprising the amino acid sequence represented by SEQ ID NO: 35, and a heavy chain variable region comprising an HCDR1 comprising the amino acid sequence represented by SEQ ID NO: 32, an HCDR2 comprising the amino acid sequence represented by SEQ ID NO: 33, and an HCDR3 comprising the amino acid sequence represented by SEQ ID NO: 34;

(g) an anti-RGMa neutralizing antibody comprising a light chain variable region comprising an LCDR1 comprising the amino acid sequence represented by SEQ ID NO: 29, an LCDR2 comprising the amino acid sequence represented by SEQ ID NO: 30, and an LCDR3 comprising the amino acid sequence represented by SEQ ID NO: 40, and a heavy chain variable region comprising an HCDR1 comprising the amino acid sequence represented by SEQ ID NO: 32, an HCDR2 comprising the amino acid sequence represented by SEQ ID NO: 33, and an HCDR3 comprising the amino acid sequence represented by SEQ ID NO: 34;

(h) an anti-RGMa neutralizing antibody comprising a light chain variable region comprising an LCDR1 comprising the amino acid sequence represented by SEQ ID NO: 29, an LCDR2 comprising the amino acid sequence represented by SEQ ID NO: 30, and an LCDR3 comprising the amino acid sequence represented by SEQ ID NO: 41, and a heavy chain variable region comprising an HCDR1 comprising the amino acid sequence represented by SEQ TD NO: 32, an HCDR2 comprising the amino acid sequence represented by SEQ ID NO: 33, and an HCDR3 comprising the amino acid sequence represented by SEQ ID NO: 34;

(i) an anti-RGMa neutralizing antibody comprising a light chain variable region comprising an LCDR1 comprising the amino acid sequence represented by SEQ ID NO: 29, an LCDR2 comprising the amino acid sequence represented by SEQ ID NO: 30, and an LCDR3 comprising the amino acid sequence represented by SEQ ID NO: 42, and a heavy chain variable region comprising an HCDR1 comprising the amino acid sequence represented by SEQ TD NO: 32, an HCDR2 comprising the amino acid sequence represented by SEQ ID NO: 33, and an HCDR3 comprising the amino acid sequence represented by SEQ ID NO: 34;

(j) an anti-RGMa neutralizing antibody comprising a light chain variable region comprising an LCDR1 comprising the amino acid sequence represented by SEQ ID NO: 29, an LCDR2 comprising the amino acid sequence represented by SEQ ID NO: 30, and an LCDR3 comprising the amino acid sequence represented by SEQ ID NO: 43, and a heavy chain variable region comprising an HCDR1 comprising the amino acid sequence represented by SEQ TD NO: 32, an HCDR2 comprising the amino acid sequence represented by SEQ ID NO: 33, and an HCDR3 comprising the amino acid sequence represented by SEQ ID NO: 34;

(k) an anti-RGMa neutralizing antibody comprising a light chain variable region comprising an LCDR1 comprising the amino acid sequence represented by SEQ ID NO: 29, an LCDR2 comprising the amino acid sequence represented by SEQ ID NO: 30, and an LCDR3 comprising the amino acid sequence represented by SEQ ID NO: 44, and a heavy chain variable region comprising an HCDR1 comprising the amino acid sequence represented by SEQ ID NO: 32, an HCDR2 comprising the amino acid sequence represented by SEQ ID NO: 33, and an HCDR3 comprising the amino acid sequence represented by SEQ ID NO: 34; and

(l) an anti-RGMa neutralizing antibody comprising a light chain variable region comprising an LCDR1 comprising the amino acid sequence represented by SEQ ID NO: 29, an LCDR2 comprising the amino acid sequence represented by SEQ ID NO: 30, and an LCDR3 comprising the amino acid sequence represented by SEQ ID NO: 45, and a heavy chain variable region comprising an HCDR1 comprising the amino acid sequence represented by SEQ TD NO: 32, an HCDR2 comprising the amino acid sequence represented by SEQ ID NO: 33, and an HCDR3 comprising the amino acid sequence represented by SEQ ID NO: 34.

6. An agent for preventing or treating pain symptoms in neuromyelitis optica, comprising a RGMa inhibiting substance.

7. The preventive or therapeutic agent according to claim 6, wherein the RGMa inhibiting substance is an anti-RGMa neutralizing antibody.

8. The preventive or therapeutic agent according to claim 7, wherein the anti-RGMa neutralizing antibody is a humanized antibody.

9. The preventive or therapeutic agent according to claim 7 or 8, wherein the anti-RGMa neutralizing antibody is an antibody recognizing the amino acid sequence selected from SEQ ID NOS: 16, 36, 37, 38 and 39.

10. The preventive or therapeutic agent according to any one of claims 7 to 9, wherein the anti-RGMa neutralizing antibody is an antibody selected from the following (a) to (1):

(a) an anti-RGMa neutralizing antibody comprising a light chain variable region comprising an LCDR1 comprising the amino acid sequence represented by SEQ ID NO: 5, an LCDR2 comprising the amino acid sequence represented by SEQ ID NO: 6, and an LCDR3 comprising the amino acid sequence represented by SEQ ID NO: 7, and a heavy chain variable region comprising an HCDR1 comprising the amino acid sequence represented by SEQ ID NO: 8, an HCDR2 comprising the amino acid sequence represented by SEQ ID NO: 9, and an HCDR3 comprising the amino acid sequence represented by SEQ ID NO: 10;

(b) an anti-RGMa neutralizing antibody comprising a light chain variable region comprising an LCDR1 comprising the amino acid sequence represented by SEQ ID NO: 11, an LCDR2 comprising the amino acid sequence represented by SEQ ID NO: 12, and an LCDR3 comprising the amino acid sequence represented by SEQ ID NO: 13, and a heavy chain variable region comprising an HCDR1 comprising the amino acid sequence represented by SEQ ID NO: 14, an HCDR2 comprising the amino acid sequence represented by SEQ ID NO: 15, and an HCDR3 comprising an amino acid sequence comprising SFG;

(c) an anti-RGMa neutralizing antibody comprising a light chain variable region comprising an LCDR1 comprising the amino acid sequence represented by SEQ ID NO: 17, an LCDR2 comprising the amino acid sequence represented by SEQ ID NO: 18, and an LCDR3 comprising the amino acid sequence represented by SEQ TD NO: 19, and a heavy chain variable region comprising an HCDR1 comprising the amino acid sequence represented by SEQ ID NO: 20, an HCDR2 comprising the amino acid sequence represented by SEQ ID NO: 21, and an HCDR3 comprising the amino acid sequence represented by SEQ ID NO: 22;

(d) an anti-RGMa neutralizing antibody comprising a light chain variable region comprising an LCDR1 comprising the amino acid sequence represented by SEQ ID NO: 23, an LCDR2 comprising the amino acid sequence represented by SEQ ID NO: 24, and an LCDR3 comprising the amino acid sequence represented by SEQ TD NO: 25, and a heavy chain variable region comprising an HCDR1 comprising the amino acid sequence represented by SEQ ID NO: 26, an HCDR2 comprising the amino acid sequence represented by SEQ TD NO: 27, and an HCDR3 comprising the amino acid sequence represented by SEQ ID NO: 28;

(e) an anti-RGMa neutralizing antibody comprising a light chain variable region comprising an LCDR1 comprising the amino acid sequence represented by SEQ ID NO: 29, an LCDR2 comprising the amino acid sequence represented by SEQ TD NO: 30, and an LCDR3 comprising the amino acid sequence represented by SEQ ID NO: 31, and a heavy chain variable region comprising an HCDR1 comprising the amino acid sequence represented by SEQ ID NO: 32, an HCDR2 comprising the amino acid sequence represented by SEQ ID NO: 33, and an HCDR3 comprising the amino acid sequence represented by SEQ ID NO: 34;

(f) an anti-RGMa neutralizing antibody comprising a light chain variable region comprising an LCDR1 comprising the amino acid sequence represented by SEQ ID NO: 29, an LCDR2 comprising the amino acid sequence represented by SEQ ID NO: 30, and an LCDR3 comprising the amino acid sequence represented by SEQ ID NO: 35, and a heavy chain variable region comprising an HCDR1 comprising the amino acid sequence represented by SEQ ID NO: 32, an HCDR2 comprising the amino acid sequence represented by SEQ ID NO: 33, and an HCDR3 comprising the amino acid sequence represented by SEQ ID NO: 34;

(g) an anti-RGMa neutralizing antibody comprising a light chain variable region comprising an LCDR1 comprising the amino acid sequence represented by SEQ ID NO: 29, an LCDR2 comprising the amino acid sequence represented by SEQ ID NO: 30, and an LCDR3 comprising the amino acid sequence represented by SEQ ID NO: 40, and a heavy chain variable region comprising an HCDR1 comprising the amino acid sequence represented by SEQ ID NO: 32, an HCDR2 comprising the amino acid sequence represented by SEQ ID NO: 33, and an HCDR3 comprising the amino acid sequence represented by SEQ ID NO: 34;

(h) an anti-RGMa neutralizing antibody comprising a light chain variable region comprising an LCDR1 comprising the amino acid sequence represented by SEQ ID NO: 29, an LCDR2 comprising the amino acid sequence represented by SEQ ID NO: 30, and an LCDR3 comprising the amino acid sequence represented by SEQ ID NO: 41, and a heavy chain variable region comprising an HCDR1 comprising the amino acid sequence represented by SEQ ID NO: 32, an HCDR2 comprising the amino acid sequence represented by SEQ TD NO: 33, and an HCDR3 comprising the amino acid sequence represented by SEQ ID NO: 34;

(i) an anti-RGMa neutralizing antibody comprising a light chain variable region comprising an LCDR1 comprising the amino acid sequence represented by SEQ ID NO: 29, an LCDR2 comprising the amino acid sequence represented by SEQ TD NO: 30, and an LCDR3 comprising the amino acid sequence represented by SEQ ID NO: 42, and a heavy chain variable region comprising an HCDR1 comprising the amino acid sequence represented by SEQ ID NO: 32, an HCDR2 comprising the amino acid sequence represented by SEQ ID NO: 33, and an HCDR3 comprising the amino acid sequence represented by SEQ ID NO: 34;

(j) an anti-RGMa neutralizing antibody comprising a light chain variable region comprising an LCDR1 comprising the amino acid sequence represented by SEQ ID NO: 29, an LCDR2 comprising the amino acid sequence represented by SEQ ID NO: 30, and an LCDR3 comprising the amino acid sequence represented by SEQ ID NO: 43, and a heavy chain variable region comprising an HCDR1 comprising the amino acid sequence represented by SEQ ID NO: 32, an HCDR2 comprising the amino acid sequence represented by SEQ ID NO: 33, and an HCDR3 comprising the amino acid sequence represented by SEQ ID NO: 34;

(k) an anti-RGMa neutralizing antibody comprising a light chain variable region comprising an LCDR1 comprising the amino acid sequence represented by SEQ ID NO: 29, an LCDR2 comprising the amino acid sequence represented by SEQ ID NO: 30, and an LCDR3 comprising the amino acid sequence represented by SEQ ID NO: 44, and a heavy chain variable region comprising an HCDR1 comprising the amino acid sequence represented by SEQ ID NO: 32, an HCDR2 comprising the amino acid sequence represented by SEQ ID NO: 33, and an HCDR3 comprising the amino acid sequence represented by SEQ ID NO: 34; and

(l) an anti-RGMa neutralizing antibody comprising a light chain variable region comprising an LCDR1 comprising the amino acid sequence represented by SEQ ID NO: 29, an LCDR2 comprising the amino acid sequence represented by SEQ ID NO: 30, and an LCDR3 comprising the amino acid sequence represented by SEQ ID NO: 45, and a heavy chain variable region comprising an HCDR1 comprising the amino acid sequence represented by SEQ ID NO: 32, an HCDR2 comprising the amino acid sequence represented by SEQ TD NO: 33, and an HCDR3 comprising the amino acid sequence represented by SEQ ID NO: 34.

11. A method of preventing or treating acute phase neuromyelitis optica, or pain symptoms in neuromyelitis optica, which comprises administration of an effective dose of a RGMa inhibiting substance to a mammal in need of treatment.

12. The preventive or therapeutic method according to claim 11, wherein the RGMa inhibiting substance is an anti-RGMa neutralizing antibody.

13. Use of a RGMa inhibiting substance in the manufacture of an agent for preventing or treating acute phase neuromyelitis optica.

**Fig. 1**

**Fig. 2**

**Fig. 3**

Fig. 4

**Fig. 5**

Fig. 6

Fig. 7

## INTERNATIONAL SEARCH REPORT

| International application No. |
| --- |
| PCT/JP2020/049004 |

A. CLASSIFICATION OF SUBJECT MATTER
A61K 45/00(2006.01)i; A61K 39/395(2006.01)i; A61P 25/02(2006.01)i; A61P
25/04(2006.01)i; A61P 27/02(2006.01)i; C07K 16/28(2006.01)i; C07K
16/46(2006.01)i; C12N 15/13(2006.01)i
FI:     A61K45/00 ZNA; A61K39/395 D; A61K39/395 N; A61P25/02 101;
        A61P27/02; A61P25/04; C07K16/28; C12N15/13; C07K16/46

According to International Patent Classification (IPC) or to both national classification and IPC

B. FIELDS SEARCHED

Minimum documentation searched (classification system followed by classification symbols)
A61K45/00; A61K39/395; A61P25/02; A61P25/04; A61P27/02; C07K16/28;
C07K16/46; C12N15/13

| Documentation searched other than minimum documentation to the extent that such documents are included in the fields searched | |
| --- | --- |
| Published examined utility model applications of Japan | 1922–1996 |
| Published unexamined utility model applications of Japan | 1971–2021 |
| Registered utility model specifications of Japan | 1996–2021 |
| Published registered utility model applications of Japan | 1994–2021 |

Electronic data base consulted during the international search (name of data base and, where practicable, search terms used)
JSTPlus/JMEDPlus/JST7580 (JDreamIII); CAplus/MEDLINE/EMBASE/BIOSIS/WPIDS
(STN); PubMed; nature.com

C. DOCUMENTS CONSIDERED TO BE RELEVANT

| Category* | Citation of document, with indication, where appropriate, of the relevant passages | Relevant to claim No. |
| --- | --- | --- |
| X | HARADA, Kana et al., "Inhibition of RGMa alleviates symptoms in a rat model of neuromyelitis optica", Scientific Reports, 08 January 2018, vol. 8, https://doi.org/10.1038/s41598-017-18362-2, abstract, fig. 1 | 1–2, 6–7, 11–13 |
| Y | abstract, fig. 1 | 3–5, 8–10 |
| Y | WO 2016/175236 A1 (MITSUBISHI TANABE PHARMA CORPORATION) 03 November 2016 (2016-11-03) claims 3, 7, 14, 17 | 1–13 |

☒ Further documents are listed in the continuation of Box C.      ☒ See patent family annex.

| | |
| --- | --- |
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 16 February 2021 (16.02.2021) | 02 March 2021 (02.03.2021) |

| Name and mailing address of the ISA/ | Authorized officer |
| --- | --- |
| Japan Patent Office | |
| 3-4-3, Kasumigaseki, Chiyoda-ku, | |
| Tokyo 100-8915, Japan | Telephone No. |

Form PCT/ISA/210 (second sheet) (January 2015)

**INTERNATIONAL SEARCH REPORT**

| International application No. |
| --- |
| PCT/JP2020/049004 |

C (Continuation). DOCUMENTS CONSIDERED TO BE RELEVANT

| Category* | Citation of document, with indication, where appropriate, of the relevant passages | Relevant to claim No. |
| --- | --- | --- |
| Y | 山下俊英，多発性硬化症に対する新規抗体治療薬の開発，平成 28 年度 委託研究開発成果報告書，18 May 2017, https://www.amed.go.jp/content/files/jp/houkoku_h28/0105016/h27_089.pdf, page 2, paragraph [0003], lines 9-12, (YAMASHITA, Toshihide, "Development of a new antibody treatment drug for multiple sclerosis", Achievement report of Commissioned R & D 2016) | 1-13 |
| A | MOTHE, Andrea J. et al., "RGMa inhibition with human monoclonal antibodies promotes regeneration, plasticity and repair, and attenuates neuropathic pain after spinal cord injury", Scientific Reports, 05 September 2017, vol. 7, https://doi.org/10.1038/s41598-017-10987-7, entire text, all drawings | 1-13 |
| A | TANABE, Shogo et al., "Inhibiting repulsive guidance molecule-a suppresses secondary progression in mouse models of multiple sclerosis", Cell Death & Disease, 17 October 2018, vol. 9, Article No. 1061, https://doi.org/10.1038/s41419-018-1118-4, entire text, all drawings | 1-13 |

Form PCT/ISA/210 (continuation of second sheet) (January 2015)

**INTERNATIONAL SEARCH REPORT**
Information on patent family members

International application No.

PCT/JP2020/049004

| Patent Documents referred in the Report | Publication Date | Patent Family | Publication Date |
|---|---|---|---|
| WO 2016/175236 A1 | 03 Nov. 2016 | US 2018/0100012 A1 claims 3, 7, 14, 17 EP 3290441 A1 CN 107531791 A KR 10-2017-0138565 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2005087268 A
- WO 2009106356 A
- WO 2013112922 A
- WO 2016175236 A
- WO 2005035586 A
- WO 200231140 A
- WO 0061739 A
- US 6737056 B
- US 7297775 B
- US 7317091 B
- US 4737456 A
- JP 4506458 W
- JP 2912618 B
- JP 62296890 A
- JP 4504365 W
- JP 7509137 W
- WO 9425585 A
- JP 6500233 W

**Non-patent literature cited in the description**

- *Lancet,* 2004, vol. 364, 2106-2112
- *J Exp Med,* 2005, vol. 202, 473-477
- *Jpn. J. Clin. Immunol.,* 2012, vol. 35 (2), 129-135
- *Journal of the Neurological Sciences,* 2011, vol. 306, 183-187
- *Neurology,* 2006, vol. 66, 1485-1489
- *Lancet Neurol,* 2007, vol. 6, 805-815
- *Curr Treat Options Neurol,* 2010, vol. 12, 244-255
- *Neurology,* 1999, vol. 53, 1107-1114
- *Magn Reson Med Sci,* 2008, vol. 7, 55-58
- *Tohoku J Exp Med,* 2008, vol. 215, 55-59
- *Neurology,* 2004, vol. 63, 1081-1083
- *Mult Sclr,* 2007, vol. 13, 128-132
- *Neuron,* 1990, vol. 5, 735-743
- *Philos. Trans. R. Soc. Lond. B Biol. Sci.,* 2006, vol. 361, 1513-29
- *Biochem. Biophys. Res. Commun.,* 2009, vol. 382, 795-800
- *Curr. Opin. Neurobiol.,* 2007, vol. 17, 29-34
- *J. Cell Biol.,* 2006, vol. 173, 47-58
- *Scientific Reports,* 2018, vol. 8 (34), 1-9
- **KABAT et al.** Sequences of Proteins of Immunological Interest. US Department of Health and Human Services, 1987
- Current Protocols in Molecular Biology. John Wiley & Sons, 1987
- Antibodies: A Laboratory Manual. Cold Spring Harbor Laboratory, 1988
- **KÖHLER ; MILSTEIN et al.** *Nature,* 1975, vol. 256, 495
- *Nucleic Acids Research,* 1986, vol. 14, 1779
- *The Journal of Biological Chemistry,* 1982, vol. 257, 1516
- *Cell,* 1980, vol. 22, 197
- **HASHIGUCHI SHUHEI et al.** *Seikagaku,* 2010, vol. 82 (8), 710
- **D. J. KING.** Applications and Engineering of Monoclonal Antibodies. T.J. International Ltd, 1998
- Monoclonal Antibody-Based Therapy of Cancer. Marcel Dekker Tnc, 1998
- **CHARI et al.** *Cancer Res.,* 1992, vol. 152, 127
- **LIU et al.** *Proc Natl Acad Sci USA.,* 1996, vol. 93, 8681
- *Nature Genetics,* 1994, vol. 7, 13-21
- *Nature Genetics,* 1997, vol. 15, 146-156
- *Nature,* 1994, vol. 368, 856-859
- **FLANAGAN et al.** *Ann Neurol.,* March 2016, vol. 79 (3), 437-47
- **WINGERCHUK et al.** *Neurology,* 2015, vol. 8582, 177-189
- **KUROSAWA K et al.** *Acta Neuropathol Commun.,* 2015, vol. 3, 82
- **TANABE S ; FUJITA Y ; TKUMA K ; YAMASHITA T.** Inhibiting repulsive guidance molecule A suppresses secondary progression in mouse models of multiple sclerosis. *Cell Death Dis,* 2018, vol. 9 (11), 1061
- **CHAPLAN, S.R. ; BACH, F.W. ; POGREL, J.W. ; CHUNG, J.M. ; YAKSH, T.L.** Quantitative assessment of tactile allodynia in the rat paw. *J. Neurosci. Methods,* 1994, vol. 53, 55-63